(19) Europäisches Patentamt
European Patent Office
Office européen des brevets

(11) EP 0 994 177 A2

(12) **EUROPEAN PATENT APPLICATION**

(43) Date of publication:
**19.04.2000 Bulletin 2000/16**

(51) Int Cl.[7]: **C11C 5/00**, A01N 25/20, A61L 9/03

(21) Application number: **99307692.6**

(22) Date of filing: **29.09.1999**

(84) Designated Contracting States:
**AT BE CH CY DE DK ES FI FR GB GR IE IT LI LU MC NL PT SE**
Designated Extension States:
**AL LT LV MK RO SI**

(30) Priority: **13.10.1998 US 169995**

(71) Applicant: **INTERNATIONAL FLAVORS & FRAGRANCES INC.**
**New York New York 10019 (US)**

(72) Inventors:
- **Bornstein, Debra Marla**
  **Cranford, New Jersey 07016 (US)**
- **Lopes, Isabel Christina**
  **Livingston, New Jersey 07039 (US)**
- **Lindauer, Jerome I.**
  **Hillsdale, New Jersey 07642 (US)**
- **Keane, Peter J.**
  **Bridgewater, New Jersey 08807 (US)**
- **Falabella, Elizabeth**
  **Point Pleasant, New Jersey 08742 (US)**
- **Dews, Allison**
  **Manalapan, New Jersey 07726 (US)**
- **Schreck, Lisa T.**
  **Tinton Falls, New Jersey 07724 (US)**

(74) Representative: **Richardson, Kate**
**Forrester & Boehmert,**
**Franz-Joseph Strasse 38**
**80801 München (DE)**

(54) **Multifunctional and/or multi-aroma imparting, multisectional, multiwick candles; kits for preparing same; processes for preparing same and processes for using same**

(57) Described are vertically-disposed, vertically prismatically-sectioned candles with each section containing a separate wick and each section having a different function (or purpose) and/or different fragrance from at least one of its adjacent sections; processes for producing such vertically-disposed, vertically prismatically-sectioned, multifragranced and/or multifunctional candles; and kits for the creation of such multifragranced and/or multifunctional candles, comprising a multiplicity of alternative wick-containing sections having different functions and/or fragrances. Also described are processes for imparting a multiplicity of fragrances and/or a multiplicity of functional vapors to the environment surrounding the candles by means of igniting same. The different functions include fragrancing; insect repelling; aroma therapy; air freshening; and malodor coverage. The prismatic candle sections may be individually, differently colored and may be individually opaque or clear; with the clarity, opaqueness and particular color being indicative of a particular function (purpose) and/or fragrance.

FIG 1A

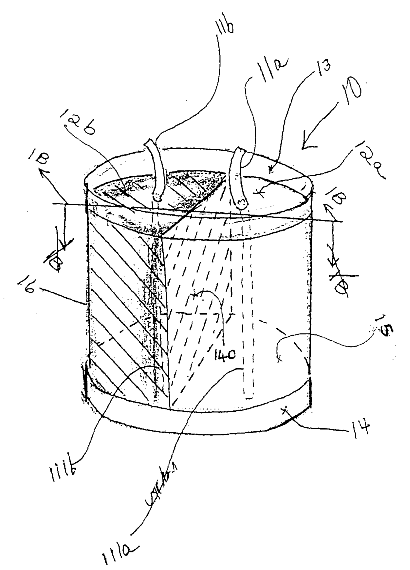

EP 0 994 177 A2

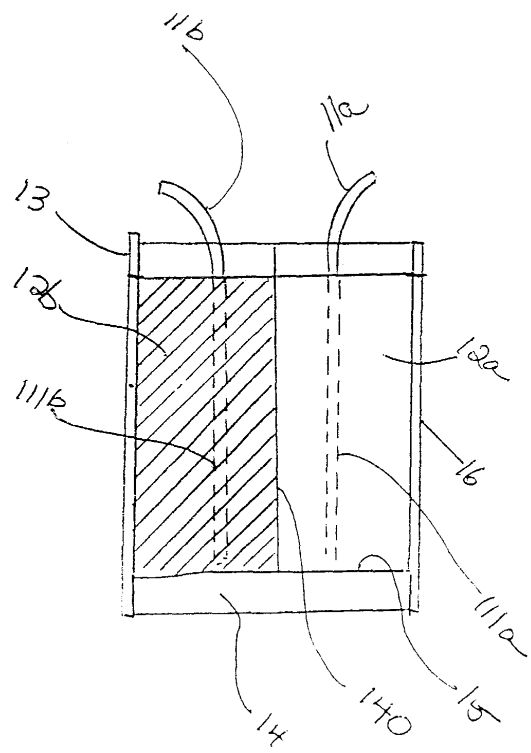


FIG 1B

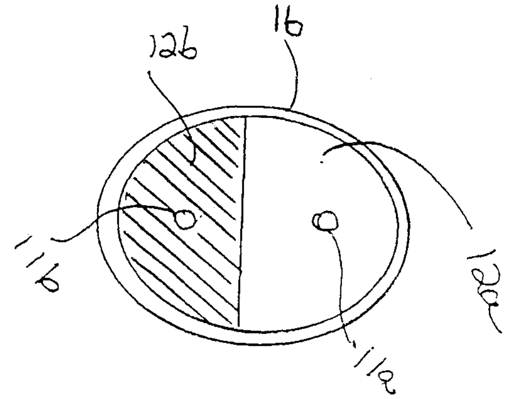


FIG 1C

## Description

## BACKGROUND OF THE INVENTION

Our invention concerns vertically-disposed, vertically-sectioned candles which are in the shape of prisms, cylinders, conical frustum sections, elliptical cylinders and the like. Each of the candle sections within the scope of our invention contains a separate wick, and each section has a different function or purpose and/or different fragrance from at least one of its adjacent sections. Each of the sections can be fused to one another to form the entire candle, and each of the sections may be prismatic or may be a section of a cylinder, elliptical cylinder or conical frustum. Our invention is also directed to kits for the creation of such multifragranced and/or multifunctional candles, and these kits comprise a multiplicity of alternative wick-containing sections having different functions and/or fragrances. These wick-containing sections are easily fused together to one another to form the entire candle. The kits can also comprise the raw materials for making the sections, for example, a wax composition, a fragrance composition and a wick for each section together with a mold for each section.

In the household products area, the use of candles for fragrancing, repelling insects, air freshening, aroma therapy and malodor coverage is highly sought after. Quietly burning candles provide a practical way of imparting into the environment such functions in a controllably, releasable manner. A need exists, however, for imparting into the environment tailor-made fragrance compositions using such candles and vapor compositions which include the other functional products as mentioned above, to wit:

insect repelling;
aroma therapy;
air freshening; and
malodor coverage.

Fragrance-providing candles are well known in the prior art. Thus, Fredericks, U.S. Letters Patent No. 3,175,876 issued on March 30, 1965, discloses a scent-yielding candle composed of a wax body, a plain cotton wick and scent-producing ingredients contained in a dried application of a sugar and a paste-forming carbonaceous substance. Thus, for example, Fredericks discloses the use of a mixture of powdered cocoa and maple sugar for the paste-forming mixture.

Gardner, et al, U.S. Letters Patent No. 4,099,976 issued on July 11, 1978, discloses an incense product, including a plurality of incense beads on a single wick. Gardner, U.S. Letters Patent No. 4,334,853 issued on June 15, 1982, went one step further and created a plurality of incense beads, including beads of different fragrances threaded in a stack on a carrier rod.

Driscoll, U.S. Letters Patent 2,323,804 issued on July 6, 1943, discloses a scent-imparting candle composed of paraffin wax and a heavy, viscous citronella oil.

Lindauer, U.S. Letters Patent No. 4,449,987 issued on May 22, 1984, describes candle compositions which may be opaque or transparent or pastel shaded, which are adapted to incorporate compositions which are both perfumes and insect repellents, without flashing during burning, composed of a mixture of (a) a hydrocarbon wax; or (b) a thermoplastic polyamide resin formed from linoleic acid polymerized with a polyamine compound taken together with an alkanol amide or an alkanol amine and a stearic acid compound; or (c) a straight chain aliphatic amide in combination with light mineral oil anti alcohol, taken further together with a mixture of methyl heptone, coumarin and indole.

However, nothing in the prior art takes advantage of the use of multiple candle sections, each containing their own individual wicks, with each section being fused together and with each section containing a different functional material and/or different fragrance. Thus, the prior art has never taught, either explicitly or implicitly, the concept of mixing functional products, including mixing fragrances in the atmosphere above a burning candle by use of different candle sections, each having different fragrances; or, for example, a candle section having a fragrance and another candle section fused to it having an insect-repelling function. Surprisingly, the creation of such a candle and the flexibility of a kit for variable creations has given rise to unexpected, unobvious and advantageous results in: fragrancing the environment surrounding the burning candle; aroma therapy; insect repellency; air freshening; as well as malodor coverage.

The prior art contains disclosures wherein several candle sections have been fused togther, but nothing in the prior art concerning the fusion of such candle sections discloses our invention, either explicitly or implicitly. Thus, U.S. Letters Patent No. 4,555,231 issued on November 26, 1985 discloses a unity candle, including two separate half sections with separate wicks on the adjoining surfaces of the two half sections; and as the half sections are moved into registered engagement, the flames from the separate wicks merge into a single flame of increased size and intensity, which melts the wax of the candle to fuse the half sections together. However, nothing is disclosed in U.S. Letters Patent No. 4,555,231 concerning different functional materials in the candle sections.

Hess, U.S. Letters Patent No. 3,744,956 issued on July 10, 1973, discloses a decorative wax candle, which includes initially separate wax inserts having colored, sculptured, relief surfaces that are directed outwardly toward the peripheral surface of the candle and which colored, sculptured, relief surface are visible through the outer peripheral surface of the candle. However, multifunctional and multiwick, multisectional candles are not expressly or implicitly disclosed by the Hess patent, U.S. Letters Patent No. 3,744,956.

## THE INVENTION

Our invention is directed to vertically-disposed, vertically prismatically-sectioned candles with each section containing a least one separate wick and with each section having a different function (or purpose) and/or different fragrance from at least one of its adjacent sections; processes for producing such vertically-disposed, vertically prismatically-sectioned, multifragranced and/or multifunctional candles; and kits for the creation of such multifragranced and/or multifunctional candles. The kits comprise a multiplicity of alternative wick-containing sections having different functions and/or fragrances. Our invention is also directed to processes for imparting a multiplicity of fragrances and/or a multiplicity of functional vapors to the environment surrounding the candle by means of igniting same. The different functions include fragrancing; insect repelling; aroma therapy; air freshening; and malodor coverage. The prismatic candle sections may be individually, differently colored and may be individually opaque or clear; with the clarity, opaqueness and particular color being indicative of a particular function (purpose) and/or fragrance.

More particularly, our invention is directed to a vertically-disposed, vertically-sectioned candle located along a vertical **Z** axis perpendicular to an **X-Y** plane, comprising a multiplicity of vertically-disposed sections, each having one or two inner flat surfaces parallel to the **Z** axis and an outer section curved surface or at least three flat, outer rectangular surfaces connected and juxtaposed to one another to form a polygon having at least three sides in the **X-Y** plane, parallel to the **Z** axis. The entirety of the inner flat surfaces of each candle section is fully, registerably contiguous with and fused to one or two inner flat surfaces of another section, with the sum of the lengths of each section curved surface or each section outer flat surface in the **X-Y** plane being the entire circumferance of the candle in the **X-Y** plane. Each section comprises a candle base composition having intimately admixed therewith at least one functional chemical, or essential oil, with each section having:

(a) at least one separate wick; and

(b) a different fragrance from at least one of the fragrances of the section or sections adjacent thereto and/or a different function from at least one of the functions of the section or sections adjacent thereto.

Thus, for example, the candle of our invention may contain two sections, each having a separate wick; with the first section having a malodor-covering function and the second section having an insect-repelling function. Furthermore, the sections may be transparent or opaque, and in either case, the sections may have different colors. Thus, included in the practice of our invention are candles having two fused, clear sections with different colors contained therein signifying different fragrances and/or different functions.

More preferably, the candle of our invention is a multifragrance-emitting candle body article substantially in the shape of a vertically-disposed cylinder or a vertically-disposed, elliptical cylinder consisting of a plurality of from two up to six discrete, vertically-disposed, upright candle sections of equal length, registerably fused to one another substantially throughout each of their interior planar, engagement surfaces; with each section comprising a candle base composition having intimately admixed therewith and uniformly dispersed therethrough from about 0.5% by weight of the composition up to about 20% by weight of the composition of an aroma-imparting composition having an aroma profile arid chemical composition distinctly different from the aroma profile and chemical composition of at least one other candle section member of said candle body. Each candle section has a vertical **Z** axis and perpendicularly intersecting the **Z** axis, perpendicularly-positioned **Y** and **X** axes. Each of the candle sections has:

(a) an upper end substantially in the **X-Y** plane and a lower end substantially in the **X-Y** plane;

(b) each of the upper end and the lower end having a matching, substantially-discontinuous rim consisting of:

(i) two straight, intersecting edges of substantially-equal length **R** joined together at one end thereof, forming an angle θ of from 60° up to 180° with one another at the intersection thereof;

(ii) joining the non-intersecting ends of each of the straight edges a circle or ellipse segment having a length equal to about $\frac{\Pi R\theta}{180}$ with the area of each of said ends equal to about $\frac{\Pi R^2\theta}{360}$ where θ is the measure of said angle of intersection in *degrees* and **R** is the length of each of said straight edges;

(c) an exterior surface conforming to said circle or ellipse segment substantially-parallel to said **Z** axis, communicating the circle or ellipse segment of said upper end, with the circle or ellipse segment of said lower end, with the edges of each of the exterior surfaces of each candle section being juxtaposed and registerably-contiguous with the edges of at least one other candle section along the **Z** axis;

(d) interior, planar, substantially-rectangular, vertically-disposed engagement surfaces conforming to and communicating said intersecting straight edges; and

(e) at least one wick extended longitudinally from a position above said upper end to a position within said candle section proximate said lower end, substantially-parallel to the **Z** axis and substantially-perpendicular to said upper end and at a position solely wwithin said section,

whereby when said wicks are burned, the fragrances and compositions of each aroma-imparting composition are admixed in the three-space proximate the burning wicks thereby forming a third fragrance composition in the gaseous phase.

Our invention is also directed to a process for producing a multifragrance-emitting (and optionally, other functional vapor-emitting (e.g., insect-repelling, malodor coverage, aroma therapy and/or air freshening) ) candle body substantially in the shape of a vertically-disposed cylinder or vertically-disposed, elliptical cylinder or other vertically-disposed prism (e.g., a rectangular parallelapiped) comprising the steps of :

(i) separately admixing each of at least two candle base compositions with at least two aroma-imparting compositions (or, as the case may be, other functional ingredients), at least one of which has a fragrance profile and chemical composition different from each of the other aroma-imparting compositions in order to form at least two aromatized candle base compositions or an aromatized candle base composition and, for example, an insect-repelling, candle base composition;

(ii) providing a plurality of molds for separately containing said functional ingredient-containing candle base compositions (e.g., aromatized candle base compositions), vertically-disposed along the **Z** axis and being of equal, vertical length along the **Z** axis, each of which mold has an upper end in the **X-Y** plane perpendicular to the **Z** axis; a lower end in the **X-Y** plane perpendicular to the **Z** axis; and three intersecting sides parallel to the **Z** axis, said upper end and said lower end each being bounded by a rim having three intersecting segments: two intersecting straight edges forming an angle of between 60° and 180° at their respective vertices and a circle or ellipse segment intersecting the non-intersecting ends of each of the said straight edges; two of the intersecting sides being intersecting, rectangular, planar laminae communating and registerably-contiguous, with said intersecting straight edges and one vertically-disposed, curvilinear lamina communicating and registerably-contiguous with said circle or ellipse segments of said upper and lower ends and juxtaposed to and registerably-contiguous with the non-intersecting vertical edges of each of said rectangular laminae;

(iii) optionally heating each of said molds to a temperature of between 0°C and 10°C greater than the melting point of said functional product-containing candle base composition (e.g., aromatized candle base compositions);

(iv) causing said functional product-containing candle base compositions to be in a molten state and healing the resulting molten composition to a temperature of between 0°C and 10°C greater than the melting point of said functional product-containing candle base composition;

(v) providing a number of candle wicks equivalent to or greater than the number of molds provided, each of which wick has a length **L** along the **Z** axis approximately equal to that of each of the molds;

(vi) placing at least one of the said provided candle wicks in a vertically-disposed manner into each of said molds;

(vii) filling each of the wick-containing molds with a provided molten, functional product-containing (e.g., aromatized candle base composition;

(viii) cooling the resulting wick-containing, functional product-containing candle base composition, contained in said molds;

(ix) removing the functional product-containing candle body sections from each of the molds; and

(x) fusing each of the resulting functional product-containing candle body sections to one another by means of placing a rectangular planar lamina of one functional product-containing candle body section against a rectangular planar lamina of a second functional product-containing candle body section at a temperature and pressure greater than the greatest fusion temperature of the functional product-containing candle body sections for a period of time $\tau$ in order to enable the aromatized candle body sections or aromatized and insect repellent candle body sections to be fused together whereby a multifragrance or a fragrance and insect repellent-emitting candle body substantially in the shape of a vertically-disposed cylinder or elliptical cylinder is formed.

More specifically, our invention covers a process for imparting a blend of at least two different fragrances (e. g., a rose fragrance and a cinnamon fragrance) into a three-space (volume) comprising the steps of:

(i) igniting each of the wicks of a multifragrance-emitting candle body as defined and formulated, supra; and

(ii) placing the candle body within the three-space for a period of time **P**, whereby the three-space is permeated with a blend of at least two different fragrances (e.g., rose and cinnamon combined) simultaneously.

The aforementioned candle body article of our invention may contain in one embodiment of our invention from about 0.5% by weight up to about 1.5% by weight of an additive, which is, in the alternative or in combination:

(i) phthalic anhydride;

(ii) succinic anhydride;

(iii) maleic anhydride;

(iv) mixtures of stearic acid and at least 50% by weight of phthalic anhydride;

(v) mixtures of stearic acid and at least 50% by weight of succinic anhydride; and

(vi) mixtures of stearic acid and at least 50% by weight of maleic anhydride.

The use of these materials aid in the even-burning of the candle body and thus even distribution of fragrance into the atmosphere surrounding the candle.

Another embodiment of our invention comprises a candle body article as described, supra, and as set forth in detail in the Detailed Description of the Drawings, infra, wherein at least one of the candle sections is composed of a clear candle composition containing, in the alternative, one of the compositions:

(i) dibenzylidene sorbitol; or

(ii) a clear gel comprising about 80-99% by weight of a hydrocarbon and from about 1 up to about 20% by weight of a gelling agent compatible with the hydrocarbon, described in detail, infra.

Another embodiment of our invention covers a candle containing at least one candle section having an insect-repelling function where the insect-repelling function is created as a result of at least one of the following materials:

(i) geraniol;

(ii) citronella oil;

(iii) KOAVONE® (trademark of International Flavors & Fragrances Inc.) (the acetylated dimer of isoamylene); or

(iv) VIOLIFF® (4-cyclooctenyl-ethyl carbonate).

Another embodiment of our invention covers a multisectioned prismatic candle as described, supra, which is a multipurpose-emitting candle body article substantially in the shape of a vertically-disposed cylinder or vertically-disposed elliptical cylinder, consisting of a plurality of from 2 up to 6 discrete, vertically-disposed, upright candle sections of equal length, fused to one another substantially throughout each of their respective, interior planar, engagement surfaces; with each section comprising a candle base composition having intimately admixed therewith and uniformly

dispersed thereghrough from about 0.5% by weight of the composition up to about 20% by weight of the composition of a volatile, single-purpose composition which is, in the alternative, an esthetically-pleasing, fragrance-imparting composition having a first purpose, an insect-repellent composition having a second purpose, a malodor-maskant composition having a third purpose, or an air freshener composition having a fourth purpose; with at least one candle member section having a purpose different from at least one other candle member section; and with each candle section having a vertical **X** axis and perpendicularly intersecting said **X** axis, perpendicularly-positioned **Y** and **Z** axes; each of said candle sections having:

(a) an upper end substantially in the **Y-Z** plane and a lower end substantially in the **Y-Z** plane;

(b) each end having a matching, substantially-discontinuous rim consisting of:

(i) two straight, intersecting edges of substantially-equal length **R** joined together at one end thereof, forming an angle θ of from 60° up to 180° with one another at the intersection thereof; and
(ii) joining the ends of each of the straight edges a circle or ellipse segment having a length equal to about $\frac{\pi R\theta}{180}$ with the area of each of said ends equal to about $\frac{\pi R^2\theta}{360}$ where θ is the measure of said angle of intersection in *degrees* and **R** is the length of each of the straight lines;

(c) an exterior surface conforming to said circle or ellipse segment substantially-parallel to said **X** axis, communicating the circle or ellipse segment of the upper end of the candle body, with the circle or ellipse segment of the lower end of the candle body and with the edges of each of the exterior surfaces of each candle section being juxtaposed and contiguous with the edges of at least one other candle section along the **Y** axis;

(d) interior, planar, substantially-rectangular, vertically-disposed engagement surfaces conforming to and communicating the intersecting straight edges; and

(e) at least one wick extended longitudinally from a position above the upper end of the candle body to a position within the candle section proximate the lower end of the candle body, which wick is substantially-parallel to the **X** axis and at a position solely within the candle section,

whereby when said wicks are burned, the compositions of each "purpose" (e.g., perfumery insect repellent and/or air freshener) composition are admixed in the three-space proximate the burning wicks thereby forming a multipurpose compostition in the gaseous phase which diffuses with the air around the burning wick.

Our invention is also directed to a process for producing a multipurpose vapor-emitting, multiwick and multisectional candle body substantially in the shape of a vertically-disposed cylinder, prism or vertically-disposed, elliptical cylinder comprising the steps of:

(i) separately admixing each of at least two candle base compositions with at least two different, single-purpose chemical compositions selected from the group consisting of an esthetically-pleasing fragrance-imparting composition having a first single purpose; an insect-repellent composition having a second single purpose; and a malodor maskant composition having a third single purpose, at least one of which chemical compositions has a first single purpose and at least a second of said chemical compositions having a second single purpose, each of said single purpose chemical compositions having a chemical composition and aroma different from each of the single-purpose compositions of an adjacent candle body section to form at least two candle base compositions containing chemical compositions having different purposes;

(ii) providing a plurality of molds for separately containing said candle base compositions, vertically-disposed along the **X** axis and being of equal, vertical length along the **x** axis, each of which molds has an upper end in the **Y-Z** plane perpendicular to the **X** axis; a lower end in the **Y-Z** plane perpendicular to the **X** axis; and three intersecting sides parallel to the **X** axis, said upper end and said lower end each being bounded by a rim having three intersecting segments: two intersecting straight edges forming an angle of between 60° and 180° at their respective vertices and a circle or ellipse segment intersecting the non-intersecting ends of each of the said straight edges; two of the intersecting sides being intersecting, rectangular, planar laminae communicating and contiguous, with said intersecting straight edged and one vertically-disposed, curvilinear lamina communicating and contiguous with said circle of ellipse segments of said upper and lower ends and juxtaposed to and contiguous with the non-intersecting vertical edges of each of said rectangular laminae;

(iii) optionally heating each of said molds to a temperature of between 0°C and 10°C greater than the respective

melting points of each of said single-purpose candle base compositions;

(iv) causing each of said candle base compositions to be in a molten state and heating the resultant molten compositions to a temperature **T** of between 0°C and 10°C greater than the melting point of said single-purpose compositions;

(v) providing a number of candle wicks equivalent to or greater than the number of molds provided, where each wick has a length **L** along the **X** axis approximately equal to the length in the **X** direction of each of the molds;

(vi) placing at least one of said provided candle wicks in a vertically-disposed manner into each of said molds;

(vii) filling each of the wick-containing molds with a provided molten, single-purpose, candle base composition which is molten;

(viii) cooling the resulting wick-containing, single-purpose candle base composition contained in said molds;

(ix) removing the single-purpose candle body sections from each of the molds; and

(x) fusing each of the resulting single-purpose candle body sections to one another by means of placing a rectangular planar lamina of one of the single-purpose candle body sections against a rectangular planar lamina of a second single-purpose candle body section at a temperature and pressure greater than the greatest fusion temperature of the single-purpose candle body sections for a period of time $\tau$ in order to enable the single-purpose candle body sections to be fused together, whereby a multipurpose candle body substantially in the shape of a vertically-disposed cylinder or elliptical cylinder is formed.

Furthermore, our invention is also directed to a process for imparting a blend of substances in the vapor phase having at least two different purposes and at least two different aromas (as set forth, supra) into a three-space (volume) comprising the steps of:

(i) igniting each of the wicks of the multipurpose, vapor-emitting candle body as set forth, supra; and

(ii) placing the resultant candle body within the three-space for a period of time $\theta$, whereby the three-space is permeated with the blend of at least two different materials in the gaseous phase having two different purposes and at least two different fragrances.

The aforementioned process and candle bodies may contain, for example, from about 0.5% up to about 1.5% by weight of an additive, which is, in the alternative:

(i) phthalic anhydride;

(ii) succinic anhydride;

(iii) maleic anhydride;

(iv) mixtures of stearic acid and at least 50% by weight of phthalic anhydride;

(v) mixtures of stearic acid and at least 50% by weight of succinic anhydride; and

(vi) mixtures of stearic acid and at least 50% by weight of maleic anhydride.

The candle body articles of our invention may include a section which is a clear candle composition, which may contain:

(a) from 0.5 up to 3.0% by weight of dibenzylidene sorbitol having the structure:

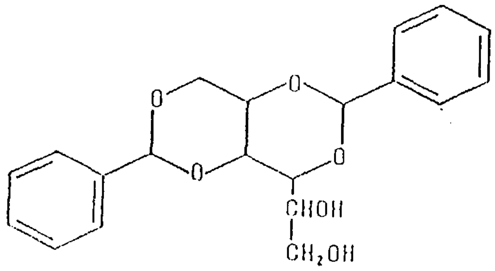

;

from 40 weight, percent up to 96.5 weight percent of at least one vegetable oil triglyceride; from 0 up to 59.5% of a compatible high molecular weight (molecular weight in the range of 250-2,500) alkanol or alkyl polyalkoxy alkanol additive acting as a partial body replacer for a portion of the vegetable oil triglyceride, the weight ratio of high molecular weight additive:triglyceride being from 0:1 up to 6:4; from 3 up to 12 weight percent of at least: one fragrance material and/or at least one insect repellent material having a flash point greater than 160°F, said composition being:

(i) water free;

(ii) $C_1$-$C_6$ alkanol free; and

(iii) $C_2$-$C_6$ alkane diol or triol free

and containing no flaring preventative agents (such as butyl stearate). The term "$C_2$-$C_6$ alkane diol and triol" is intended herein to include such compounds as ethylene glycol, 1,2-propylene glycol, 1,3-propylene glycol, 1,4-butane diol, 1,2-butane diol, 1,3-butane diol and 1,6-hexane diol.
Examples of vegetable oil triglycerides useful in producing the clear candle sections of our invention are:

safflower oil;
sesame oil;
castor oil; and
palm oil.

Examples of "compatible high molecular weight additives" useful in producing the clear candle sections of our invention are:

(i) EUTANOL® G having the structure:

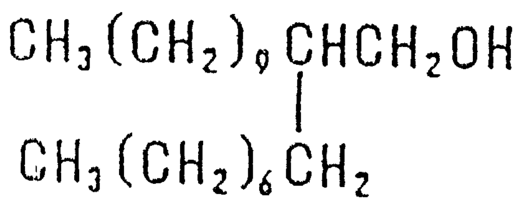

(molecular weight = 297) (a trademark of The Henkel Corporation of Minneapolis, Minnesota)

(ii) UCON HB 660® (trademark of Amerchol Corporation) having the structure:

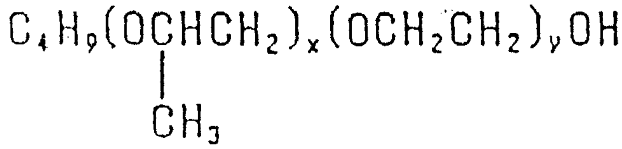

where **x** has an average value of 12 and **y** has an average value of 16 (molecular weight = 1474); and

(iii) UCON LB 1715® (trademark of Amerchol Corporation having the structure:

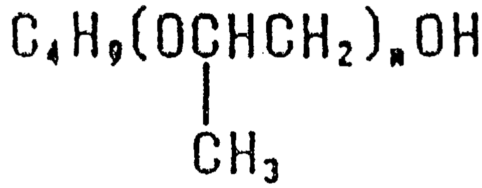

wherein **n** has an average value of 40 (molecular weight = 2394); and

(b) a clear gel comprising about 80-99 weight percent of a hydrocarbon and about 1-20 weight percent of a blend of at least two different polymer members selected from the group consisting of diblock copolymers, triblock copolymers, radial-block copolymers and multiblock copolymers, said composition including at least one diblock copolymer and at least one triblock copolymer, with said diblock and triblock polymers comprising segments of styrene monomer units and rubber monomer units (as disclosed and claimed in Elsamaloty, U.S. Letters Patent No. 5,578,089 issued on November 26, 1996, the specification for which is incorporated by reference herein).

Our invention is more preferably and specifically directed to candle body articles as set forth, supra, wherein:

(a) a first candle section has the purpose of insect repellency and at least one section comprises a candle body composition having admixed therewith at least one of the following materials:

(i) a mixture of aldehydes and ketones having the structures:

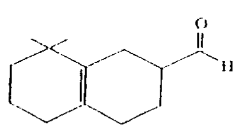 ; 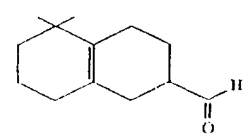 ;

and

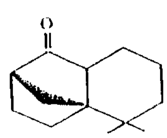

containing from 45-60 mole percent of the compound having the structure:

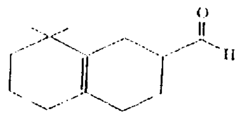 ;

from 15-20 mole percent of the compound having the structure:

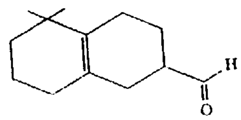 ;

and from 25-35 mole percent of the compound having the structure:

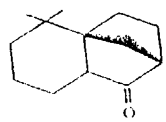

as disclosed and claimed in U.S. Letters Patent No. 5,354,783 issued on October 11, 1994, the specification for which is incorporated by reference herein;

(ii) geraniol having the structure:

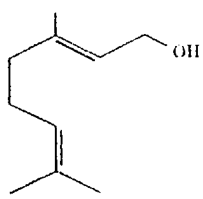 ;

(iii) a β-geraniol glucoside having the structure:

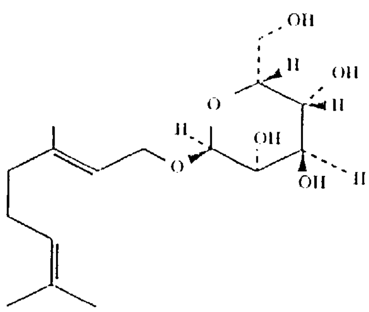 ;

(iv) a mixture of citronellol having the structure:

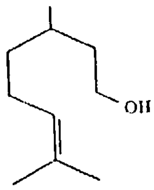

and nerol having the structure:

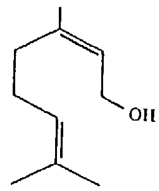

;

(v) at least one geranyloxy-1,3,2-dioxaborinane defined according to the generic structure:

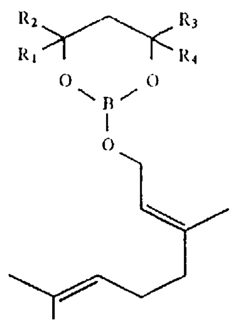

wherein $R_1$, $R_2$, $R_3$ and $R_4$ are the same or different hydrogen, methyl and ethyl including the compound having the structure:

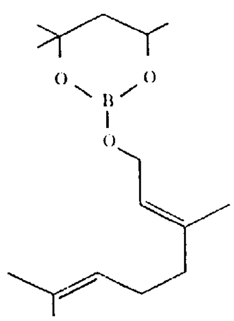

;

(vi) a digeranyloxy-dialkyl silane defined according to the structure:

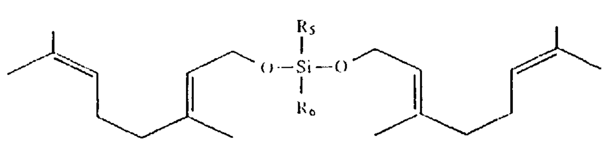

wherein $R_5$ and $R_6$ are the same or different $C_1$-$C_5$ lower alkyl including the compound having the structure:

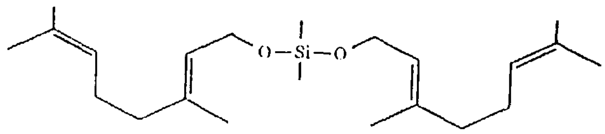

;

(vii) at least one oxymethyl cyclohexane derivative defined according to the structure:

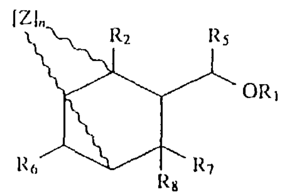

wherein **Z** is methylene; **n** is 0 or 1; each of the wavy lines represents a carbon-carbon single bond or no bond; the dashed line represents a carbon-carbon double bond or a carbon-carbon single bond; each of $R_2$, $R_5$, $R_6$, $R_7$ and $R_8$ are the same or different and each represents hydrogen or methyl; and $R_1$ represents hydrogen, acetyl or ethoxycarbonyl with the provisos:

(i) that **n** is 1 and each of the wavy lines represents a carbon-carbon single bond when $R_1$, $R_2$, and $R_6$, are each hydrogen, $R_5$, $R_7$ and $R_8$ are each methyl and the dashed line is a carbon-carbon single bond;

(ii) **n** is 0 and each of the wavy lines represents no bond when $R_5$ and $R_7$ are each hydrogen and $R_6$ and $R_8$ are each methyl;

(iii) $R_2$ is methyl only when $R_1$ is ethoxycarbonyl; and

(iv) the dashed line is a carbon-carbon single bond only when $R_1$ is hydrogen, such as the compounds having the structures:

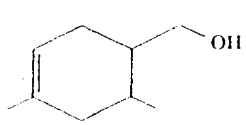

;

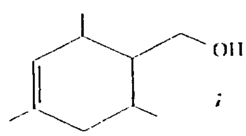

;

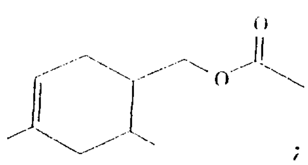

;

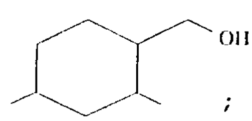

;

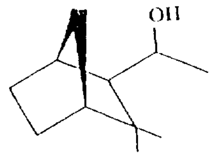 ; and 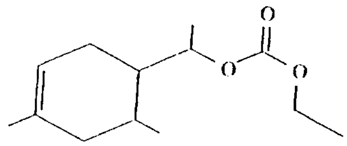

as described in U.S. Letters Patent No. 5,576,010 issued on November 19, 1996, the specification for which is incorporated by reference herein;

(viii) geraldehyde having the structure:

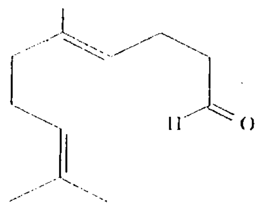

and the compound having the structure :

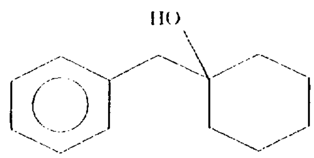

as disclosed in U.S. Letters Patent No. 5,527,768 issued on June 18, 1996, the specification for which is incorporated by reference herein; and

(b) a second of the candle sections having the purpose of aromatixing the environment surrounding the candle, which comprises a candle body composition having intimately admixed therewith at least one of the following fragrance materials:

(i) a rose fragrance containing, for example, bois de rose oil;

(ii) a cinnamon fragrance containing, for example, cassia oil;

(iii) a woody fragrance containing, for example, KOAVONE®, a mixture of compounds defined according to the structure:

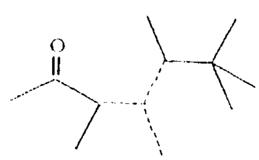

(representing a mixture wherein in each of the compounds of the mixture, one of the dashed lines represents a carbon-carbon double bond and each of the other of the dashed lines represents a carbon-carbon single bond) or KOAVOL® DH having the structure:

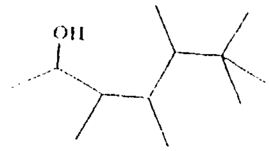

The clear candle sections and/or the opaque candle sections of our invention can also contain various colors, and production of such colored candle compositions is shown, inter alia, in U.S. Letters Patent No. 5,726,145 issued on March 10, 1998, the specification for which is incorporated by reference herein.

The clear and/or opaque candle sections of our invention may each also contain a liquid fuel for generating colored flames, for example, those which comprise ethylene glycol and a coloring compound such as boron, sodium and copper, as set forth in PCT Published Application No. 98/00480 published on January 21, 1998, the specification for which is incorporated by reference herein.

The clear and/or opaque candle sections used in the practice of our invention can also contain malodor counteractant compositions such as those disclosed in U.S. Letters Patent No. 5,683,979 issued on November 4, 1997, the specification for which is incorporated by reference herein, containing, for example, GALAXOLIDE®, a mixture of compounds having the structures:

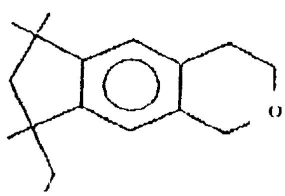 ; 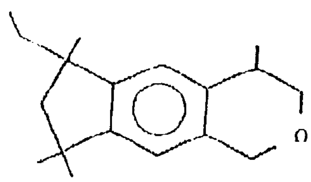 ; and 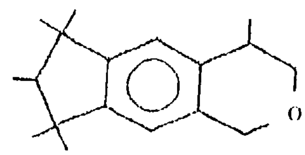

as well as the musk compound having the structure:

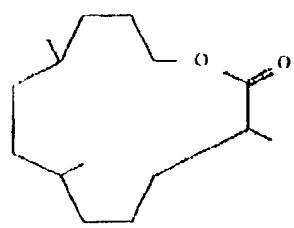

and, in addition, citrus notes, for example, mixtures of 45-65% geranial having the structure:

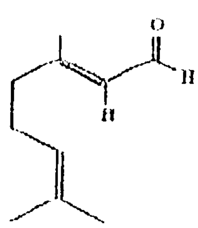

and 30-50% neral having the structure:

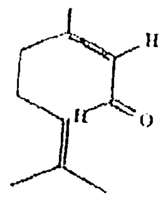

and, in addition, a mint aroma such as corn mint oil defined according to the spectra of Figures 11 and 12, described, infra, in the sections entitled "Brief Description of the Drawings" and "Detailed Description of the Drawings."

Other substances useful in formulating the opaque candle sections of our invention are ester-terminated polyamide gels as described in detail in Published PCT Application No. 98/17243, assigned to the Union Camp Corporation and published on April 30, 1998, the specification for which is incorporated by reference herein. In Published PCT Application No. 98/1/243, an ester-terminated, dimer acid-based polyamide may be blended with a solvent to form a gel. The solvent may be flammable and a wick may be added to the resulting gel so as to form a candle section. Such a solvent may be, for example, mineral oil. The ester-terminated, dimer acid-based polyamide may also be combined with an active ingredient such as a fragrance, colorant, insect repellent, insecticide, bioactive ingredient or the like in order to afford a delivery vehicle for the active ingredient. Thus, for example, EMPOL® 1008 is polymerized with ethylene diamine and esterified with stearyl alcohol to give an ester-terminated, dirner acid-based polyamide. The polymer is then combined with 50% mineral oil, heated and cooled to give it gelled body, thereby formulating one of the candle sections of our invention.

The intensity of functionalization of each of the candle sections containing at least one "purpose" ingredient is proportional to the rate of evolution of the mixture of functional ingredients into the atmosphere; which, in turn, is a function of the concentration $\mathbf{C_i}$ of the functional ingredient in each section, the rate $\mathbf{R_1}$ of gaseous diffusion and free energy of mixing **G** of each of the functional ingredients above the burning wick with each other and with the surrounding air, the rate of transport $\mathbf{R_2}$ of each of the functional ingredients from each wick into the atmosphere during the burning of each wick; which, in turn, is a function of the wick flame temperature and heat transfer from the flame to the functional ingredient; and the rate $\mathbf{R_3}$ of transport of the mixture of functional ingredients from a fluid pool (where the design of the article gives rise to such fluid pool) on the surface of the multiwick candle (as illustrated in Figure 5, described in detail, infra). Mathematical models describing such intensity of functionalization are set forth as follows:

$$I = \sum_{i=2}^{N} K_i C_i + \lambda_1 \int_0^{\theta} \sum_{j=1}^{3} \sum_{i=2}^{N} R_{ij} \, d\theta + \lambda_2 G$$

wherein **I** is the intensity of functionalization and $\mathbf{R_{ij}}$ are the rates of transport, and the symbols $\mathbf{K_i}$, $\lambda_1$ and $\lambda_2$ are constants. The rates $\mathbf{R_1}$, $\mathbf{R_2}$ and $\mathbf{R_3}$ are shown as follows:

$$R_3 = \int_0^{\theta} \sum_{i=2}^{N} D'_i \left(\nabla^2 C'_i\right) d\theta \; ; \quad R_1 = \int_0^{\theta} \sum_{i=2}^{N} D_i \left(\nabla^2 C_i\right) d\theta \; ;$$

and

$$R_2 = \int_0^{\theta} \sum_{i=2}^{N} D_i \left( \nabla C_i \left[ Q_i, T_i \right] \right) d\theta \; ;$$

and, more particularly, for a cylindrical candle, the rate $\mathbf{R_2}$ is shown as follows:

$$R_2 = \int_0^{\theta} \sum_{i=2}^{N} \frac{4D_{si}}{R_i^2} e^{\frac{-5.8D_{si}\theta}{R_i^2}} d\theta$$

wherein

$$D_{si} = \left\{ \frac{KT^{3/2}\sqrt{\sum_{i=2}^{N}\frac{1}{M_i}}}{PR_i^2} \right\} .$$

The free energy of mixing **G** is shown by the equation:

$$G = \sum_{i=2}^{N} G_i = RT \sum_{i=2}^{N} x_i \ln\left[\sum_{j=i}^{N} x_j \Lambda_{ij}\right]$$

wherein R is the gas constant, **T** is temperature and wherein **x** represents the mole fraction of each component in the mixture (taken from Reid, et al, *The Properties of Gases and Liquids,* McGraw-Hill Book Company, 1977, Chapters 4 and 8) in the foregoing equations:

$$\nabla^2 C_i = \left[\frac{\partial^2 C_i}{\partial x^2} + \frac{\partial^2 C_i}{\partial y^2} + \frac{\partial^2 C_i}{\partial z^2}\right]$$

and

$$\nabla C_i\left[Q_i, T_i\right] = \left[\frac{\partial C_i\left[Q_i, T_i\right]}{\partial x} + \frac{\partial C_i\left[Q_i, T_i\right]}{\partial y} + \frac{\partial C_i\left[Q_i, T_i\right]}{\partial z}\right] .$$

Our invention is also directed to a kit for enabling the creation of a multifunctional, composition-emitting candle body article substantially in the shape of a vertically-disposed prism, which, in the alternative, is a parallelepiped, vertically-disposed cylinder, vertically-disposed elliptical cylinder or vertically-disposed upright or inverted conical frustum having a continuous or discretely-sectioned circumference of length **C** consisting of a plurality of $\mathbf{2 \leq N \leq 6}$, separate, discrete, vertically-disposed upright candle sections of equal length capable of being permanently fused to one another substantially throughout each of their respective interior, planar engagement surface; each section comprising:

(a) candle base composition having intimately admixed terewith and uniformly dispersed terethrough different, individual, sectional, functional compositions each of which is selected from the group consisting of:

(i) fragrance composition;
(ii) insect-repelling compositon;
(iii) malodor-covering composition; and
(iv) air freshening compositions; and

(b) a vetically-disposed wick within the confines of said section and traversing substantially the entire length of the section,

each of which sections has (1) a vertical planar face which substantially, completely, contiguously fits one face of a section designed to be adjacent thereto and (2) at least one outer rim segment having a length $\mathbf{L_i}$ wherein:

$$\sum_{i=2}^{N} L_i = C \ .$$

The candle body article may be in the shape of a tetrahedron having **N** sections and **N** sides with each section having a rim segment of length **L**; or the candle body article to be completed may in the shape of a cylinder having N sections, each having an equal rim segment of length: $\mathbf{L_i} = \frac{\pi \mathbf{R} \theta}{180}$ wherein **R** is the cylinder radius and θ is the vertex angle of the interior, planar engagement surfaces of each section and wherein:

$$\sum_{i=2}^{N} L_i = 2\pi R \ .$$

Each section of the kit may contain a different fragrance such as:

(i) a rose fragrance as described in detail, supra;
(ii) a cinnamon fragrance as described in detail, supra;
(iii) a musk fragrance; and
(iv) a woody fragrance.

Thus, the kit can contain a multitude of sections whereby two up to six of these sections may be combined in a **MIX & MATCH™** (trademark of International Flavors & Fragrances Inc.) group of candle sections that may be put together in various manners to create a mix of desired fragrances above the burning candle ultimately formed using the individual members of the kit. This is shown in more particularity in the Detailed Description of the Drawings, infra.

## BRIEF DESCRIPTION OF THE DRAWINGS

Figure 1A sets forth a cutaway perspective view of a candle body article of our invention contained within a container having a wall extending upwardly beyond the candle sections of the candle body of our invention. The candle body of Figure 1A contains two candle body sections of substantially equal volume, each having an individual wick.
Figure 1B is a cutaway side elevation view of the article of Figure 1A taken along line 1B-1B of Figure 1A.
Figure 1C is a top view of the article of Figure 1A.
Figure 2A is a perspective view of a second embodiment of the candle body article of our invention contained within a container having a side wall extending above the upper part of the candle body of our invention. The candle body of our invention contains four sections of substantially equal volume, with each of the four sections having an individual wick.
Figure 2B is a cutaway side elevation view of the article of Figure 2A taken along line 2B-2B of Figure 2A.
Figure 2C is a top view of the article of Figure 2A.
Figure 3A is a perspective view of a third embodiment of the candle body of our invention wherein the candle body is contained within a transparent container having six sections vertically juxtaposed to one another, the candle body being in the shape of an elliptical cylinder. Each section of the candle body contains one individual wick.
Figure 3B is a cutaway side elevation view of the article of Figure 3A taken along line 3B-3B of Figure 3A.

Figure 3C is a top view of the article of Figure 3A.

Figure 3D is a top plan view of a modified version of the third embodiment of the candle body of our invention wherein the candle body is contained within a transparent container having five sections, of mutually different angular extent, vertically juxtaposed to one another, the candle body being in the shape of an elliptical cylinder. Each section of the candle body contains one individual wick.

Figure 4A is a block flow, schematic diagram of a process for producing the candle body shown in Figure 1A as well as the process of using that candle body without the inclusion of the candle body in the transparent container.

Figure 4B is a schematic, block flow diagram showing the process fur producing the candle body of Figure 1A contained in a transparent container and also showing the ultimate use of the candle body of Figure 1A contained in the transparent container.

Figure 5 is another schematic diagram showing the last steps of the process for producing the candle body of Figure 1A contained within a transparent container.

Figure 6 is a schematic flow diagram showing a preferred process for producing the candle body of Figure 3A contained in a transparent container ready for use.

Figure 7A sets forth a cutaway perspective diagram of a fourth embodiment of the candle body article of our invention in the shape of a rectangular parallelepiped containing two sections in the shape of triangular parallelepipeds, each section being juxtaposed to the other section, each section containing an individual wick.

Figure 7B is a top view of the article of Figure 7A.

Figure 8A is a fifth embodiment of the candle body of our invention in the shape of a triangular parallelepiped and containing three sections, each in the shape of a triangular parallelepiped, with each section being contiguous with the next section and with each section containing an individual wick. Two of the sections are shown to be composed of a clear candle body composition.

Figure 8B is a top view of the candle body of Figure 8A.

Figure 9A is a sixth embodiment of the candle body of our invention in the shape of a triangular parallelepiped containing two sections contiguous with one another, with each section containing an individual wick; and with each section being in the shape of right triangular parallelepiped; and with each section being contiguous with one another at their respective altitude sections; and with each section containing an individual wick.

Figure 9B is a top view of the candle body of Figure 9A.

Figure 10 is a schematic block flow diagram showing the various steps in a process wherein a clear, transparent, controllably-burnable candle section for use in the candle article of our invention is produced.

Figure 10A is a cutaway, schematic elevation view of a clear candle section useful in the practice of our invention produced according to the process shown in Figure 10.

Figures 11 and 12 are the GLC profiles of corn mint oil as specifically described in U.S. Letters Patent No. 5,683,979 issued on November 4, 1997, the specification for which is incorporated by reference herein; which corn mint oil is used in candle body compositions for the purpose of malodor coverage (the corn mint oil being taken together with GALAXOLIDE® or other musk odorant and a citrus note such as a mixture of geranial and neral).

Figure 13A is a perspective view of a seventh embodiment of the candle body of our invention in the shape of a hexagonal parallelepiped and containing six sections, each in the shape of a triangular parallelepiped, with each section being continguous with the next section, and with each section containing an individual wick. Two of the sections are shown to be composed of a clear candle body composition.

Figure 13B is a cutaway side elevation view of the article of figure 13A taken along the line 13B-13B of figure 13A.

Figure 13C is a side view of the article of figure 13A.

## DETAILED DESCRIPTION OF THE DRAWINGS

Referring to Figures 1A, 1B and 1C, the candle body article indicated by reference numeral 10 is composed of two sections, 12a and 12b, which, when placed together at common surface 140, form an elliptical cylinder. Section 12a contains wick 111a having an upper portion 11a. Section 12b contains wick 111b which has an upper portion 11b. When both sections 12a and 12b are contiguous with one another and juxtaposed at common surface 140, they fit into transparent cylinder (e.g., composed of glass) having side wall 16 and having an upper end 13 which extends vertically above the upper surface of the candle body. The bottom of the cylinder is indicated by reference numeral 14, and the resting surface for the candle body on the bottom of the cylinder is indicated by reference numeral 15.

Referring to Figure 2A, the candle body article indicated by reference numeral 200 has four juxtaposed sections indicated by reference numerals 22a, 22b, 22c and 22d. Section 22a contains wick 20a. Section 22b contains wick 20b. Section 22c contains wick 20c. Section 22d contains wick 20d. Section 22a is contiguous with section 22b and has a common, vertical, rectangular face 240a. Section 22b is immediately adjacent section 22c and has a common, contiguous face 240b. Section 22c is contiguous with section 22d and has a common, contiguous face 240c. Section

22d is contiguous with section 22a and has a common, contiguous face 240d. The four sections all meet at a contiguous, vertical intersection 240. The four sections, when together as a candle body, are contained in a transparent cylinder (e.g., glass) indicated by reference numeral 26. The upper portion of the transparent container cylinder, which extends above the surface of the candle body, is indicated by reference numeral 23. The lower portion of wick 20a is indicated by reference numeral 121a. The lower portion of wick 20b is indicated by reference numeral 121b. The lower portion of wick 20c is indicated by reference numeral 121c. The lower portion of wick 20d is indicated by reference numeral 121d. The bottom of the containment cylinder is indicated by reference numeral 24, and the surface on which the candle body rests is indicated by reference numeral 25. When the candle is burned, the liquid containing the different fragrances and other useful materials, e.g., insect repellents, air fresheners and malodor covering compositions, mix above the candle body in the proximity of the sidewall portion indicated by reference numeral 23. Vapors are evolved from the liquid into the atmosphere together with vapors evolving from burning wicks 20a, 20b, 20c and 20d. These vapors diffuse in with the air and form a combined fragrance together with, if desired, other materials, e.g., insect repellents.

Referring to Figure 3A, six candle body sections are fused together to form an elliptical cylinder. The six candle bodies are indicated by reference numerals 32a, 32b, 32c, 32d, 32e and 32f, respectively. Section 32a contains wick 131a having upper portion 30a. Section 32b contains wick 131b having upper portion 30b. Section 32c contains wick 131c having upper portion 30c. Section 32d contains wick 131d having upper portion 30d. Section 32e contains wick 131e having upper, portion 30e. Section 32f contains wick 131f having upper portion 30f. The wicks are contained in a cylinder indicated by reference numeral 36. The candle body rests on the bottom of the cylinder indicated by reference numeral 34. The upper portion of the cylinder (preferably transparent, composed of, for example, glass), which extends beyond the upper surface of the candle body, is indicated by reference numeral 33. When the candle is burned, liquid in the proximity of that portion of the cylinder indicated by reference numeral 33 is formed, wherein fragrance from each of sections 32a, 32b, 32c, 32d, 32e and 32f is admixed. At the same time, fragrance and other material from the upper portions of the wicks 30a, 30b, 30c, 30d, 30e and 30f evaporates into the environment surrounding the candle during the burning, and the vapors are admixed to form a combined fragrance resulting from the use of the **MIX & MATCH™** Kit as described, supra.

Similarly, figure 3D shows five candle body sections, of mutually different angular extent, fused together to form an elliptical cylinder. The five candle body sections are indicated by reference numerals 350a, 350b, 350c, 350d and 350e. Each candle body section 350 a-e has a wick, each wick having an upper portion 351 a-e respectively. The five fused sections are contained in a cylinder 352, which is similar to cylinder 36 described above.

Referring to Figure 4A, a first fragrance such as a rose fragrance contained in container 41 is admixed with a candle base contained in container 42. Thus, the fragrance is passed through line 43 past control valve 44 into mixing vessel 48. Candle base from container 42 is passed through line 45 past control valve 46 into mixing vessel 48. In mixing vessel 48, the candle base and the first fragrance is admixed using mixing means 70. Optionally, a first color (e.g., a red color) is passed into the mixing vessel from vessel 51. Heat is supplied through heating means 49 so that the fragrance and candle base are thoroughly admixed in a liquid state. The resulting liquid containing first fragrance, candle base and, optionally, color, are passed through line 52 into mold 53. Initially, a first wick from location 54 is also placed in a vertical position into mold 53. The wick shown by reference numeral 11b and having a lower portion 111b is contained in a semi-cylindrical, candle body section 12b having a flat face 140b. Similarly, a second fragrance from container 57 is passed through line 60 past control valve 61 into mixing vessel 63. Simultaneously, candle base from vessel 58 is passed through line 59 past control valve 62 into the same mixing vessel 63. Optionally, color (e.g., a second color, e.g., blue) is passed from vessel 64 into mixing vessel 63. Heat is supplied using heating means (e.g., ceramic heating coil) 65. Mixing means (e.g., stirrer) is engaged (indicated by reference numeral 71), thereby mixing and fluidizing the mixture of candle base, second fragrance (e.g, cinnamon fragrance) and, optionally, second color (e.g., blue) in vessel 63. The resulting candle base-second fragrance-optional color fluid mass is then passed through line 66 into mold 67, which also contains a second wick from location 68. After the mold containing the candle base-fragrance-color (optional) composition is cooled, the candle base article, semicircular, cylindrical section is removed. The wick therein is shown by reference numeral 11a, and the flat face thereof is shown by reference numeral 140a. The semicircular section itself is shown by reference numeral 12a. The two sections are fused at contiguous face 140 where the face of section 12b (shown by reference numeral 140b) is joined to the face of section 12a (indicated by reference numeral 140a), thereby forming a cylindrical candle body indicated by reference numeral 12. The burning candle body indicated by reference numeral 10 then yields into the atmosphere above the burning candle a mixed fragrance; one from section 12d and the second fragrance from section 12b, and the mixed fragrance in the vapor phase is shown by reference numeral 56.

Figure 4B sets forth a second embodiment of the process of Figure 4A wherein the candle body 12 is incorporated into cylinder 16. The candle body incorporated into cylinder 16 is shown by reference numeral 10. As the candle burns, a liquid is formed above the top surface of the candle body indicated by reference numeral 91, and mixed fragrance vapor is evolved during the burning of the candle and is shown by reference numeral 56.

Figure 5 sets forth another embodiment of the process of our invention wherein the article of Figure 1A is produced using a transparent cylinder having a heating element affixed to the wall 16 thereof. The heating element is indicated by reference numeral 17. Thus, when the two sections 12a and 12b are introduced into cylinder 16, resting on the bottom of the cylinder indicated by reference numeral 14 and when heat is applied through heating element 17, the two sections 12a and 12b fuse.

By the same token, Figure 6 sets forth an additional embodiment of a process for producing the candle body and article shown in Figure 3. Sections 32a, 32b and 32c are all fused to form the semi-elliptical cylinder indicated by reference numeral 32g. By the same token, sections 32d, 32e and 32f are fused to form the semi-elliptical, cylindrical section 32h. Semi-elliptical, cylindrical sections 32g and 32h are Introduced into containment, transparent cylinder 36 having a bottom 34e and equipped with heating element 37. After the semi-elliptical, cylindrical sections 32g and 32h are introduced into the cylinder 36, the heating element is engaged, thereby causing the semi-elliptical, cylindrical sections 32g and 32h to fuse, thus forming the article indicated by reference numeral 300.

Referring to Figure 7A, the candle body article 700, which is is a rectangular parallelepiped, contains two triangular parallelepiped sections 701 and 702. Section 701 contains wick 707, and section 702 contains wick 708. Section 701 has a bottom 706, and section 702 has a bottom 705. Section 702 has sidewalls 704 and 703. Sections 701 and 702 have a common face where the sections are contiguous with one another and sealed to one another as indicated by reference numeral 741.

Figures 8A and 8B set forth another embodiment of a candle body of our invention, which is a triangular parallelepiped prism containing three contiguous, triangular parallelepiped sections. The candle body of Figure 8A and Figure 8B is indicated by reference numeral 800. Triangular parallelepiped section 801 contains wick 809. Section 803 contains wick 810, and section 802 contains wick 811. Wick 811 extends to the bottom of triangular parallelepiped section 802, which bottom is indicated by reference numeral 806. By the same token, the bottom of triangular parallelepiped section 803 is indicated by reference numeral 807, and the bottom of triangular parallelepiped section 801 is indicated by reference numeral 808. The outer sidewall of triangular parallelepiped 803 is indicated by reference numeral 805, and the outer sidewall of triangular parallelepiped 801 is indicated by reference numeral 804.

Figures 9A and 9B show another embodiment of a prismatic candle body of our invention, which is in the form of a triangular parallelepiped 900 having two right triangular parallelepiped sections fused to one another at the altitude section of each of the right triangular parallelepipeds. This altitude, contiguous face (or interface) is indicated by reference numeral 908 and is in the form of a rectangular plane extending from the bottom of the triangular parallelepiped 900 (indicated by reference numerals 906 and 907) to the upper face of the triangular parallelepiped. Right triangular parallelepiped section 901 contains wick 910 and has two outer faces, rectangular in shape, indicated by reference numerals 903 and 904 with the hypotenuse of section 901 indicated by reference numeral 903. Right triangular parallelepiped section 902 contains wick 909 and outer faces 905 and 911 with the hypotenuse face being indicated by reference numeral 911.

Referring to Figure 10A, Figure 10A is a clear candle section useful in the creation of a candle body such as that set forth in Figure 8 (with sections 802 and 803 thereof being clear candle body sections). The clear candle section of Figure 10A is indicated by reference numeral 423. The wick in the candle section is indicated by reference numeral 424, and the clear candle section is indicated in Figure 10A by reference numeral 421..

In producing the clear candle section of Figure 10A, a process is carried out as shown in the schematic block flow diagram of Figure 10. Dibenzylidene sorbitol having the structure:

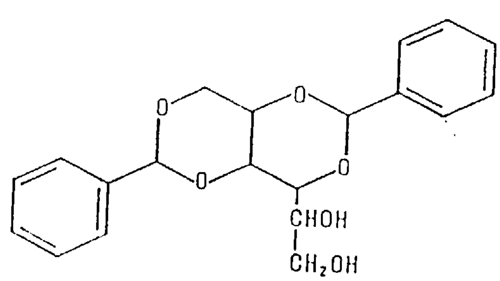

at a holding tank indicated by reference numeral 1 is passed through line 4 using pump 3 through gate valve 450 into tank 7 equipped with heater and stirrer equipped to operate from 125°C up to 185°C. Simultaneously, vegetable oil triglyceride taken together with compatible high molecular weight additive in holding vessel 2 is passed through line 6 using pump 5 through gate valve 451 into holding vessel 7. The mixing takes place for a period of about 15 minutes after which, with stirring, the mixing is carried on for a period of from about 0.1 up to about 1 hour at 125-185°C. At

the end of this period, the resulting mixture is passed through line 8 using pump 9 through gate valve 452 into cooling vessel 410. Coolant 411a/411b is passed, for example, counter-currently past the mixture from vessel 7 in cooling vessel or heat exchanger 410. The cooled material from heat exchanger 410 is passed through line 412 using pump 413 through valve 400 into vessel 416 where fragrance and/or insect repellent from vessel 431 is admixed in tank 416. The fragrance and/or insect repellent from tank 431 is passed through line 414 using pump 415 through gate valve 500. The mixing of the fragrance and/or insect repellent is carried out for a period of from 0.1 up to 1 hour in vessel 416 after which the resultant material is passed through line 417 using pump 418 through gate valve 600 into apparatus 419, which contains candle section molds supplied from location 422. The candle section molds are permitted to cool and passed into distribution center 432. The cooled candle sections are indicated by reference numeral 421. These candle sections can be fused with other candle sections to produce articles such as that indicated in Figure 8.

Similarly figures 13A, 13B and 13C show a hexagonal parallelepiped candle body 1350 containing six contiguous triangular parallelepiped sections, 1351 a-f which are fused together. Each section 1351 a-f contains a wick, each wick having an upper portion 1352 a-f.

Candles having the same features as the candles exemplified in this application, but having a different shape and/or divided into a different number of sections are also contemplated by the present invention.

The following examples are illustrative of specific embodiments of our invention and it is not intended that the invention be limited thereto

## EXAMPLE A

## INSECT REPELLENT COMPOSITION

The following insect repellent composition hereinafter indicated by the letter "A" is produced by admixing the following ingredients:

| Ingredients | Parts by Weight | Flash Point (°F) |
|---|---|---|
| P-Isopropyl cyclohexanol | 4.0 | 196°F |
| 1, 2, 3, 4, 5, 6, 7, 8-Octahydro 8,8-dimethyl-2-naphthaldehyde | 2.0 | >212°F |
| 2,4-Dimethyl cyclohexane methanol acetate | 2.0 | 192°F |

The composition and each of its individual components each have flash points >160°F.

## EXAMPLE B

The following perfume composition indicated by the letter "B" is prepared by admixing the following ingredients:

| Ingredients | Parts by Weight | Flash Point (°F) |
|---|---|---|
| Methyl-3, 6-dimethyl-β-resorcylate | 20 | 192°F |
| 2-Methyl-4-phenyl-2-butanol | 10 | >212°F |
| 10-Undecanal | 100 | 175°F |
| 1-Ethynyl cyclohexanyl acetate | 20 | 179°F |
| Oxacycloheptadecan-2-one | 80 | >212°F |
| 2,4,6-Trimethyl-3-cyclohexene-1-carboxaldehyde | 10 | 172°F |
| 2,4-Dimethyl cyclohexane methanol | 6 | 196°F |
| 2-Ethylidene-6-isopropoxy Bicyclo [2.2.1] heptane | 10 | 165°F |
| Phenyl ethyl propionate | 60 | >212°F |
| α-Terpineol | 10 | 195°F |
| 3-Methyl-5-phenyl-1-pentanol | 60 | >212°F |
| Phenethyl cyclohexyl ether | 14 | >212°F |

(continued)

| Ingredients | Parts by Weight | Flash Point (°F) |
|---|---|---|
| Benzyl acetate | 468 | 195°F |
| Myrcenyl acetate | 80 | 180°F |
| Geranyl ethyl ether | 6 | 190°F |
| 2, 3, 6-Trimethyl cyclohexen-4-yl-1-methyl ketone | 12 | 182°F |
| Cis-3-hexenyl salicylate | 42 | >212°F |
| 2,6-Dimethyl-3, 4-octadien-2-ol | 10 | 182°F |

The resulting mixture is indicated hereinafter to be perfume composition "B."
The entire composition and each of its components each has a flash point > 160°F.

**EXAMPLE IA**

45.5 Parts by weight safflower oil, 45.5 parts by weight EUTANOL® G and 1.0 parts by weight of dibenzylidene sorbitol having the structure:

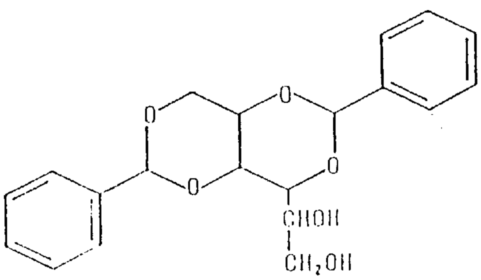

are admixed in a vessel and heated at atmospheric pressure to 160°C. With stirring, the resulting mixture is maintained at 160°C for a period of one hour. The resulting mixture is then cooled to 100°C and with stirring 8.0 parts by weight of fragrance "B" is added to the mixture. The resulting material is poured into semi-elliptical, cylindrical (as shown in Figures 1A, 1B and 1C, described, supra) sectional candle molds previously containing wicks and the sectional candle molds containing the candle composition are allowed to cool to room temperature.

**EXAMPLE IB**

**INSECT REPELLENT CANDLE SECTION**

91.0 Parts by weight castor oil are admixed with 1.25 parts by weight of dibenzylidene sorbitol having the structure:

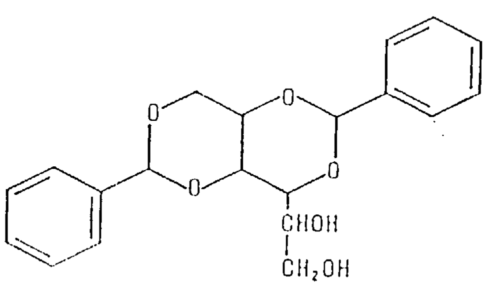

in a vessel and the contents are heated to 160°C and maintained at that temperature for a period of 0.75 hours with stirring. At the end of the 0.75 hours, the mixture is cooled to 100°C and 8.0 parts by weight of insect repellent "A"

produced according to Example "A," supra, is added to the vessel. The resulting mixture is stirred for a period of 0.1 hour. The resulting mixture is then poured into semielliptical, cylindrical, sectional candle molds having the same dimensions as the sectional molds of Example 1A, each sectional mold containing a single wick. The sectional candle molds containing the composition are cooled to room temperature.

**EXAMPLE IC**

**PRODUCTION OF INSECT REPELLENT AND FRAGRANCED CANDLE BODIES**

The candle sections produced according to Example IA and according to Example IB are fused together on their flat, vertical surfaces to form an elliptical, cylindrical candle body as shown in Figures 1A, 1B and 1C and according to Figure 4A.

The resulting candles burn in a controllable burning manner and emit a pleasant floral fragrance while burning.

Furthermore, the resulting candles have excellent insect-repelling properties. The candles are effective in preventing mosquitoes from entering a room in which two candles have been burning for 15 minutes, the said room having dimensions of 6' x 15' x 15' and having a 3' x 3' open portal adjacent a mosquito-laden swamp in the month of August in Highlands, New Jersey, Monmouth County.

The candles are clear candles, with each section being clear; the section produced according to Example IA and the section produced according to Example 1B.

In the present specification "comprise" means "includes or consists of" and "comprising" means "including or consisting of'.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

**Claims**

1. A vertically-disposed, vertically-sectioned candle located along a vertical **Z** axis perpendicular to an **X-Y** plane comprising a multiplicity of vertically-disposed sections, each having one or two inner flat surfaces parallel to the **Z** axis and an outer section curved surface parallel to the **Z** axis or at least three flat, outer rectangular surfaces connected and juxtaposed to one another to form polygons having at least three sides in the **X-Y** plane parallel to the **Z** axis; the entirety of the inner flat surfaces of each section being fully, registerably contiguous with and fused to two inner flat surfaces of another section, the sum of the lengths of each section curved surface or each section outer flat surface in the **X-Y** plane being the entire circumference of the candle in the **X-Y** plane, each section comprising a candle base composition having intimately admixed therewith at least one functional chemical and each section having:

   (a) at least one separate wick; and

   (b) a different fragrance from at least one of the fragrance or fragrances of the section or sections adjacent thereto and/or a different function from at least one of the functions of the section or sections adjacent thereto.

2. The candle of Claim 1 wherein the functions are selected from the group consisting of:

   (i) fragrancy;
   (ii) insect repelling;
   (iii) malador covering;
   (iv) air freshening; and (v) aroma therapy.

3. The candle of Claim 1 containing two sections, a first section and a second section, each section having different fragrances.

4. The candle of Claim 1 having from two up to six sections.

5. The candle of Claim 3 wherein the second section is also insect repellent.

**6.** The candle of Claim 1 containing two sections, a first section and a second section, the first section having a malodor-covering function and the second section having an insect-repelling function.

**7.** The candle of Claim 1 wherein each section has an outer appearance different from each other section.

**8.** The candle of Claim 7 wherein each section is of a different color.

**9.** The candle of Claim 7 wherein at least one section is transparent and at least one other section is opaque.

**10.** The candle of Claim 1 which is a multifragrance-emitting candle body article substantially in the shape of a vertically-disposed cylinder or vertically-disposed elliptical cylinder consisting of a plurality of from two up to six discrete, vertically-disposed, upright candle sections of equal length fused to one another substantially throughout each of their interior, planar, engagement surfaces; each section comprising a candle base composition having intimately admixed therewith and uniformly dispersed therethrough from about 0.5% by weight of the composition up to about 40% by weight of the composition of aroma-imparting compositions having an aroma profile and chemical composition distinctly different from the aroma profile and chemical composition of at least one other candle section member of said candle body; each candle section having a vertical **Z** axis is and perpendicularly intersecting said perpendicularly-positioned **Y** and **X** axes; each of said candle sections having:

(a) an upper end substantially in the **X-Y** plane and a lower end substantially in the **X-Y** plane;

(b) each end having a matching, substantially-discontinuous rim consisting of:

(i) two straight, intersecting edges of substantially-equal length **R** joined together at one end thereof, forming an angle θ of from 60° up to 180° with one another at the intersection thereof; and

(ii) joining the non-intersecting ends of each of the straight edges, a circle or ellipse segment having a length equal to about $\frac{\pi \mathbf{R} \theta}{180}$ with the area of each of said ends equal to about $\frac{\pi \mathbf{R}^2 \theta}{360}$ where θ is the measure of said angle of intersection in degrees and **R** is the length of each of the straight lines;

(c) an exterior surface conforming to said circle or ellipse segment substantially-parallel to said **Z** axis, communicating the circle or ellipse segment of said upper end of the candle body, with the circle or ellipse segment of said lower end with the edges of each of the exterior surfaces of each candle section being juxtaposed and registerably contiguous with the edges of at least one other candle section along the **Z** axis;

(d) interior, planar, substantially-rectangular, vertically-disposed engagement surfaces conforming to and communicating said intersecting straight edges; and

(e) at least one wick extended longitudinally from a position above said upper end to a position within said candle section proximate said lower end substantially-parallel to the **Z** axis and at a position solely within said section,

whereby when said wicks are burned, the fragrances and compositions of each aroma-imparting composition are admixed in the 3-space proximate the burning wicks thereby forming a third fragrance composition in the gaseous phase.

**11.** A process for producing a multifragrance-emitting and, optionally, insect-repelling candle body substantially in the shape of a vertically-disposed cylinder, or vertically-disposed, elliptical cylinder comprising the steps of:

(i) separately admixing each of at least two candle base compositions with at least two aroma-imparting compositions at least one of which has a fragrance profile and chemical composition different from each of the other aroma-imparting compositions in order to form at least two aromatized candle base compositions;

(ii) providing a plurality of molds for separately containing said aromatized candle base compositions, vertically-disposed along the **Z** axis and being of equal, vertical length along the **Z** axis, each of which molds has an upper, end in the **X-Y** plane perpendicular to the **Z** axis; a lower end in the **X-Y** plane perpendicular to the **Z** axis; and three intersecting sides parallel to the **Z** axis, said upper end and said lower end each being bounded by a rim having three intersecting segments: two intersecting straight edges forming an angle of between 60°

and 180° and their respective vertices and a circle or ellipse segment intersecting the non-intersecting ends of each of the said straight edges; two of the intersecting sides is being intersecting, rectangular, planar laminae communicating and being registerably-contiguous, with said intersecting straight edges and one vertically-disposed, curvilinear lamina communicating and contiguous with said circle or ellipse segments of said upper and lower ends and juxtaposed to and contiguous with the non-intersecting vertical edges of each of said rectangular laminae;

(iii) optionally heating each of said molds to a temperature of between 0°C and 10°C greater than the melting point of said aromatized candle base composition;

(iv) causing said molten, aromatized candle base to be in a molten state and heating the resultant composition to a temperature of between 0°C and 10°C greater than the melting point of said candle composition;

(v) providing a number of candle wicks equivalent to or greater than the number of molds provided, each of which wick has a length **L** along the **Z** axis approximately equal to that of each of the molds;

(vi) placing at least one of said provided candle wicks in a vertically-disposed manner into each of said molds;

(vii) filling each of the wick-containing molds with a provided molten, aromatized candle base composition;

(viii) cooling the resulting wick-containing, aromatized candle base compositions contained in said molds;

(ix) removing the aromatized candle body sections from each of the molds; and

(x) fusing each of the resulting aromatized candle body sections to one another by means of placing a rectangular planar lamina of one aromatized candle body section against a rectangular planar lamina of a second aromatized candle body section at a temperature and pressure greater than the greatest fusion temperature of the azomatized candle body sections for a period of time $\tau$ in order to enable the aromatized candle body sections to be fused together, whereby a multifragrance-emitting candle body substantially in the shape of a vertically-disposed cylinder or elliptical cylinder is formed.

**12.** A process for imparting a blend of at least two different fragrances into a 3-space (volume) comprising the steps of:

(i) igniting each of the wicks of a multifragrance-emitting candle body of Claim 1; and

(ii) placing said candle body within said 3-space for a period of time $\theta$, whereby said 3-space is permeated with said blend of at least two different fragrances.

**13.** A process for imparting a blend of at least two different fragrances into a 3-space (volume) comprising the steps of:

(i) igniting each of the wicks of a multifragrance-emitting candle body of Claim 2; and

(ii) placing said candle body within said 3-space for a period of time $\theta$, whereby said 3-space is permeated with said blend of at least two different fragrances.

**14.** The candle body article of Claim 1 wherein each of the candle sections contains from about 0.5% up to about 1.5% by weight of an additive selected from the group consisting of:

(i) phthalic anhydride;

(ii) succinic anhydride;

(iii) maleic anhydride;

(iv) mixtures of stearic acid and at least 50% by weight of phthalic anhydride;

(v) mixtures of stearic acid and at least 50% by weight of succinic anhydride; and

(vi) mixtures of stearic acid and at least 50% by weight of maleic anhydride.

**15.** The candle body article of Claim 1 wherein one of the candle sections is a clear candle composition containing a composition selected from the group consisting of:

(i) dibenzylidene sorbitol; and

(ii) a clear gel comprising from about 80 up to 99% by weight of a hydrocarbon and from 1 up to 20% by weight of a polymeric material.

**16.** The candle body article of Claim 1 wherein one of the candle sections has an insect-repelling function.

**17.** A kit for enabling the creation of a multifunctional composition-emitting candle body article substantially in the shape of a vertically-disposed prism, which is, in the alternative, a vertically-disposed parallelepiped; a vertically-disposed cylinder; a vertically-disposed, elliptical cylinder; or a vertically-disposed, upright or inverted conical frustum having a continuous or discretely-sectioned circumference of length **C** consisting of: **2** $\leq$**N** $\leq$**6,** separate, discrete, vertically-disposed, upright candle section of equal length capable of being permanently fused to one another substantially throughout each of their respective interior, planar engagement surfaces; each section comprising a candle base composition having intimately admixed therewith and uniformly dispersed therethrough different individual, sectional, functional compositions, each of which is selected from the group consisting of:

(i) fragrance compositions;
(ii) insect-repelling compositions;
(iii) malodor-covering compositions;
(iv) air freshening compositions; and
(v) aroma therapy compositions

and a vertically-disposed wick within the confines of said section and traversing substantially the entire length of said section; each of which sections has:

(i) a vertical planar face which substantially, completely, contiguously fits one face of a section designed to be adjacent thereto; and
(ii) at least one outer rim segment having a lenght **L** wherein:

$$\sum_{i=2}^{N} L_i = C \ .$$

**18.** The kit of Claim 17 wherein the candle body article is fragrance-emitting, and each section contains a fragrance composition of matter having a chemical composition and aroma different *in kind* from any other section.

**19.** The kit of Claim 17 wherein the candle body article is in the shape of a tetrahedron having **N** sections and **N** sides, each having a rim segment of length **L**.

**20.** The kit of Claim 17 in which the candle body article to be completed is in the shape of a cylinder having **N** sections, each having an equal. rim segment of length: $\mathbf{L_i} = \frac{\pi \mathbf{R} \theta}{\mathbf{180}}$ wherein **R** is the cylinder radius and θ is the vertex angle of the interior, planar engagement surfaces of each ssection and wherein:

$$\sum_{i=2}^{N} L_i = 2\pi R \ .$$

**21.** A candle or vapour delivery device divided into two or more sections, wherein at least one section contains a functional material and wherein at least one other section contains a different functional material.

**22.** A candle or vapour delivery device according to Claim 21, wherein the at least two sections containing different

functional materials are adjacent one another.

**23.** A candle or vapour delivery device according to Claim 21 or 22, wherein the at least two sections containing different functional materials are each provided with one or more wicks.

**24.** A kit for preparing a candle or vapour delivery device according to any one of Claims 21 to 23, which kit comprises two or more quantities of a candle base composition, at least two different functional materials, two or more wicks, and a mold for each differently shaped section.

**25.** A process for preparing a candle or vapour delivery device according to any one of Claims 21 to 23, which process comprises the steps of:

(a) providing two or more quantities of a candle base composition,
(b) admixing at least one quantity of candle base composition with a functional material,
(c) separately admixing at least one further quantity of candle base composition with a different functional material,
(d) molding each candle base composition into a separate section, and
(e) fusing the resulting sections together.

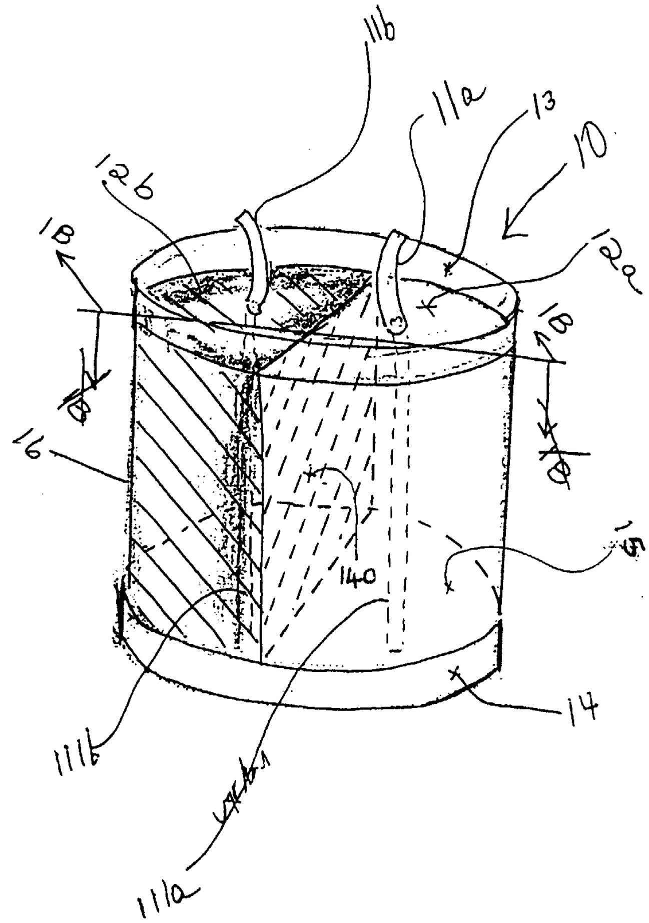


FIG 1A

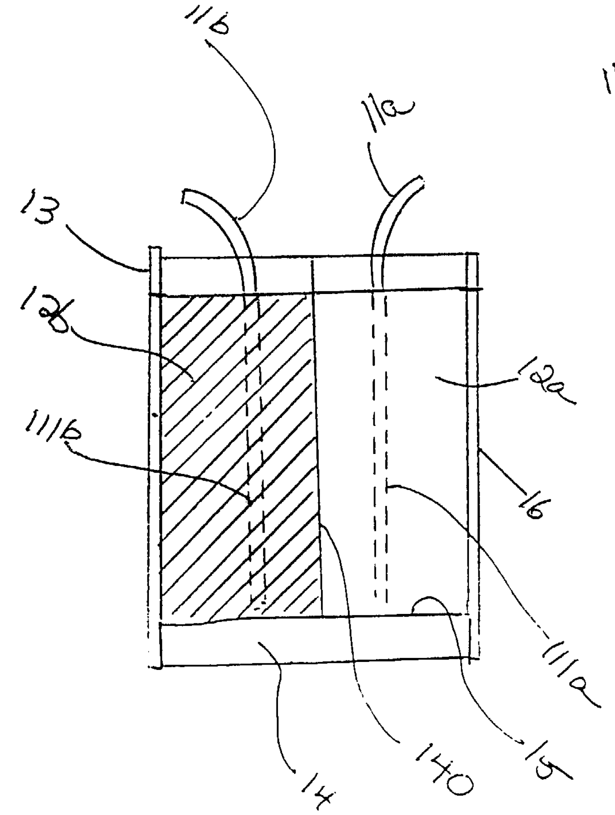


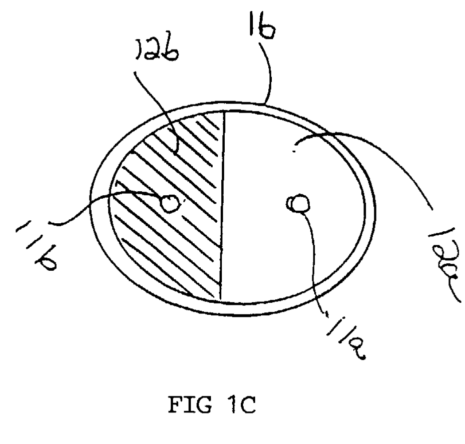


FIG 1C

FIG 1B

FIG 2A

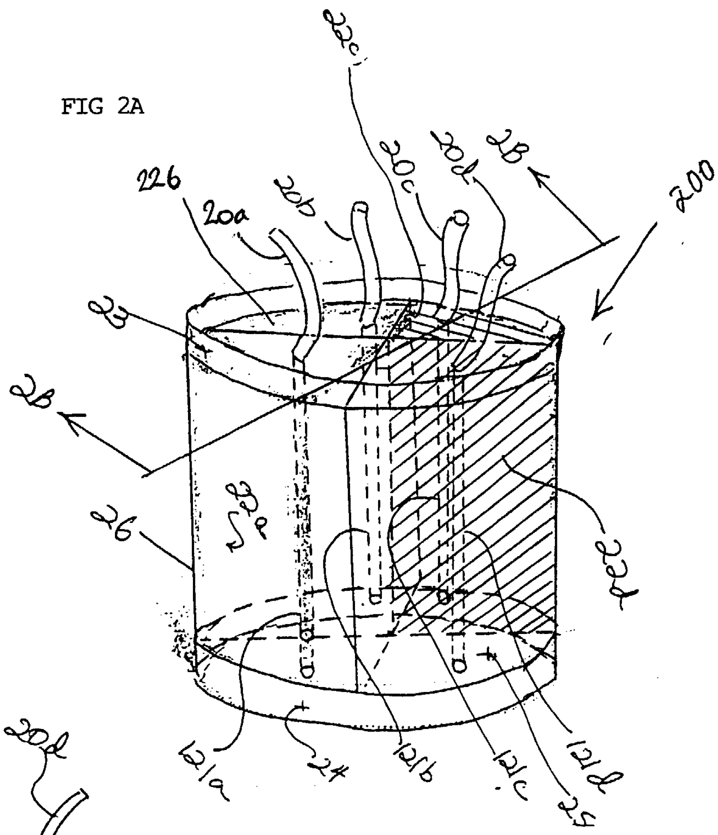


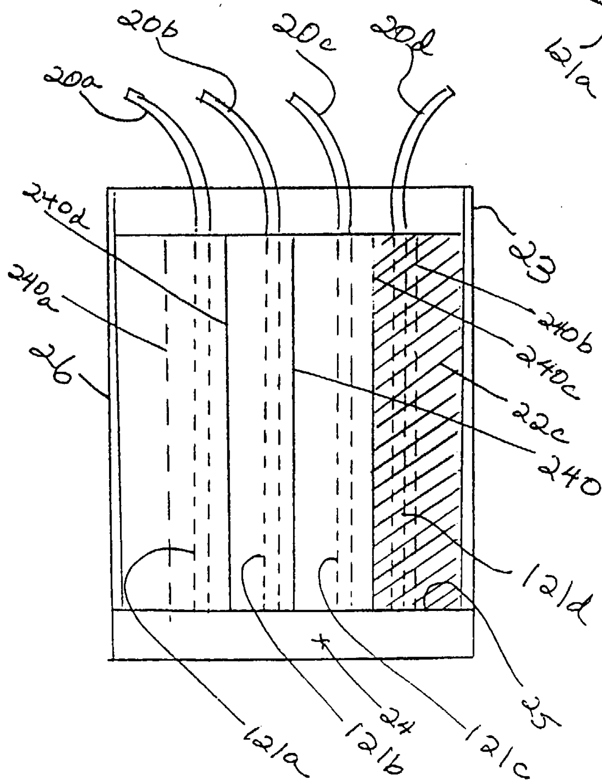


FIG 2B

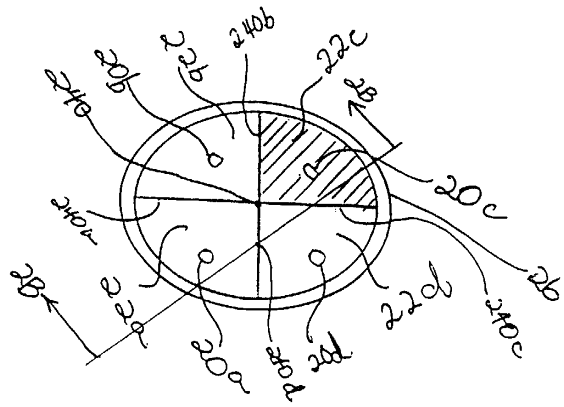


FIG 2C

FIG 3A

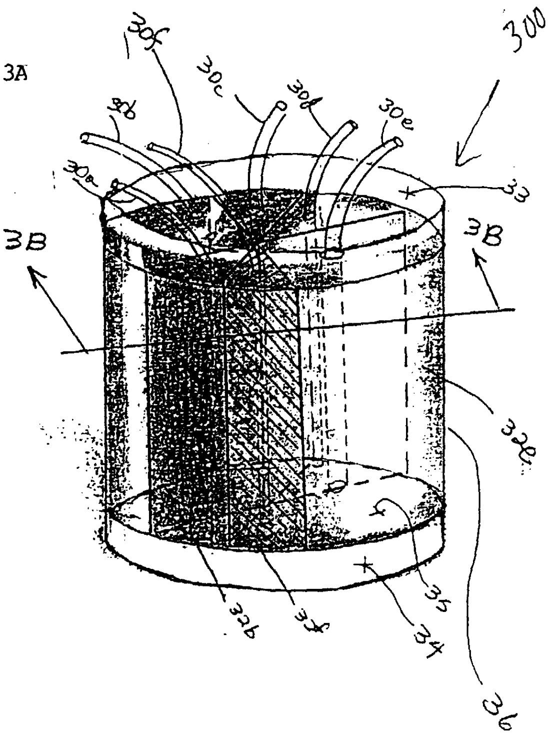


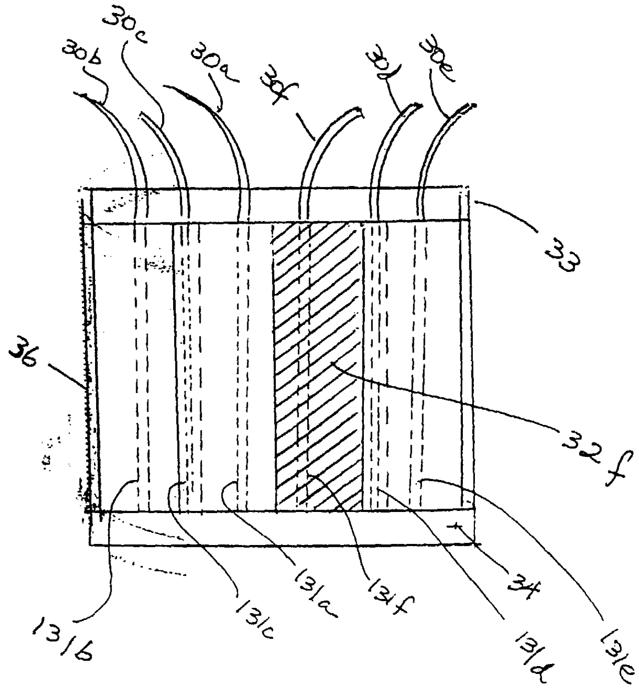


FIG 3B

FIG 3C

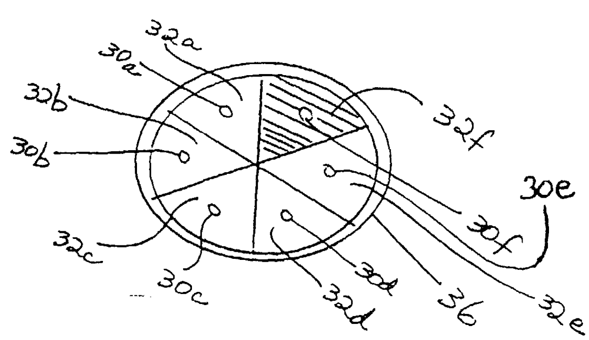


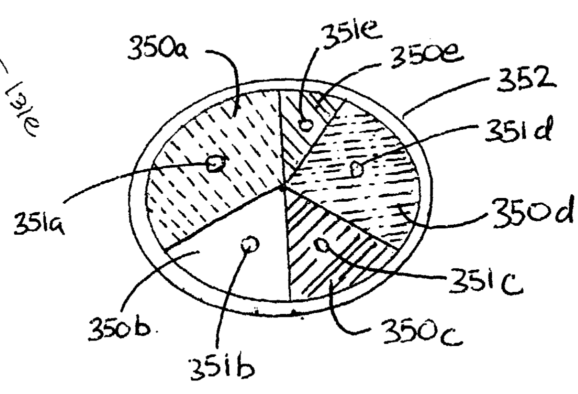


FIG 3D

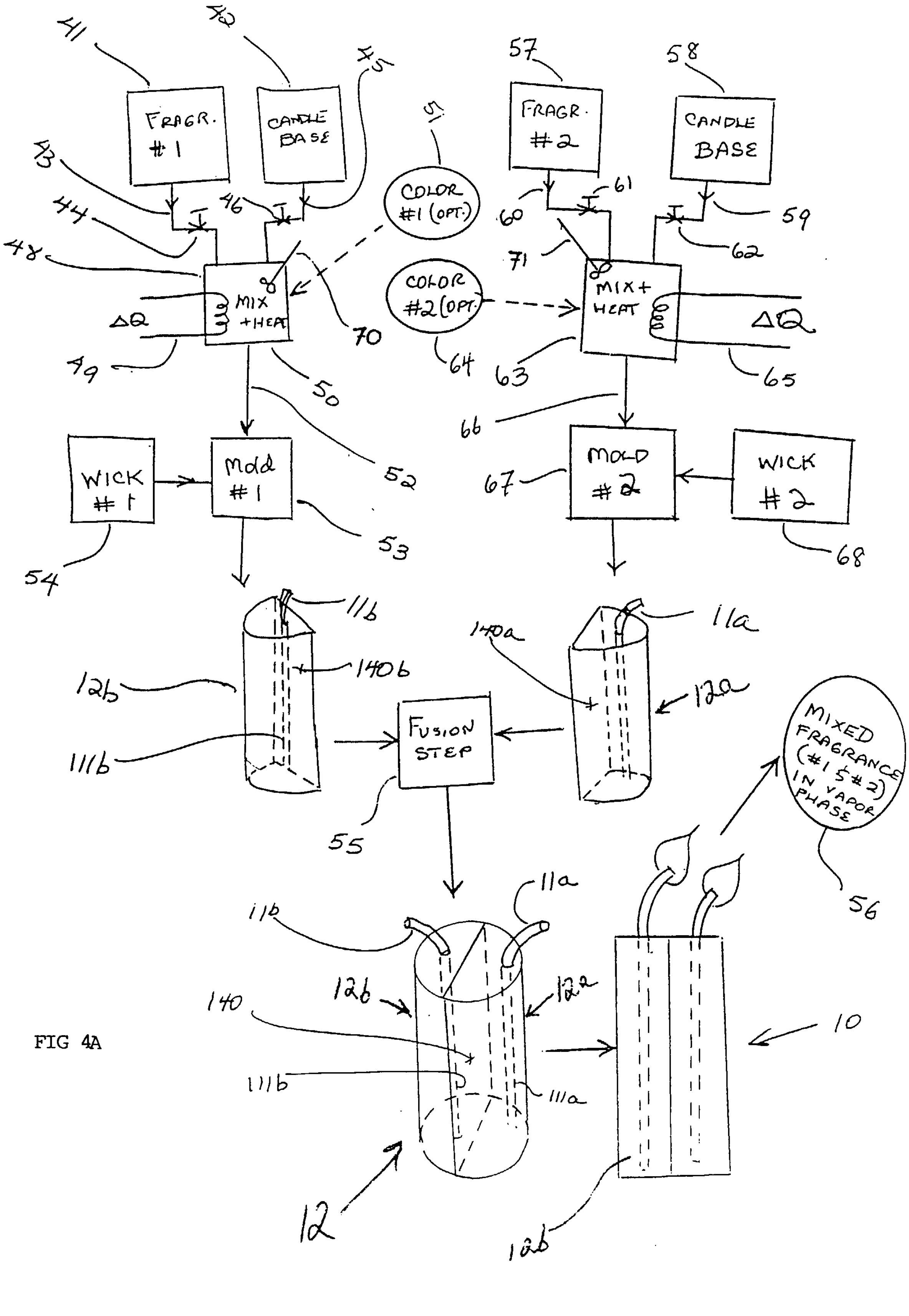
FRAGR. #1
CANDLE BASE
COLOR #1 (OPT.)
FRAGR. #2
CANDLE BASE
COLOR #2 (OPT.)
MIX +HEAT
MIX + HEAT
ΔQ
ΔQ
WICK #1
Mold #1
MOLD #2
WICK #2
FUSION STEP
MIXED FRAGRANCE (#1 & #2) IN VAPOR PHASE

FIG 4A

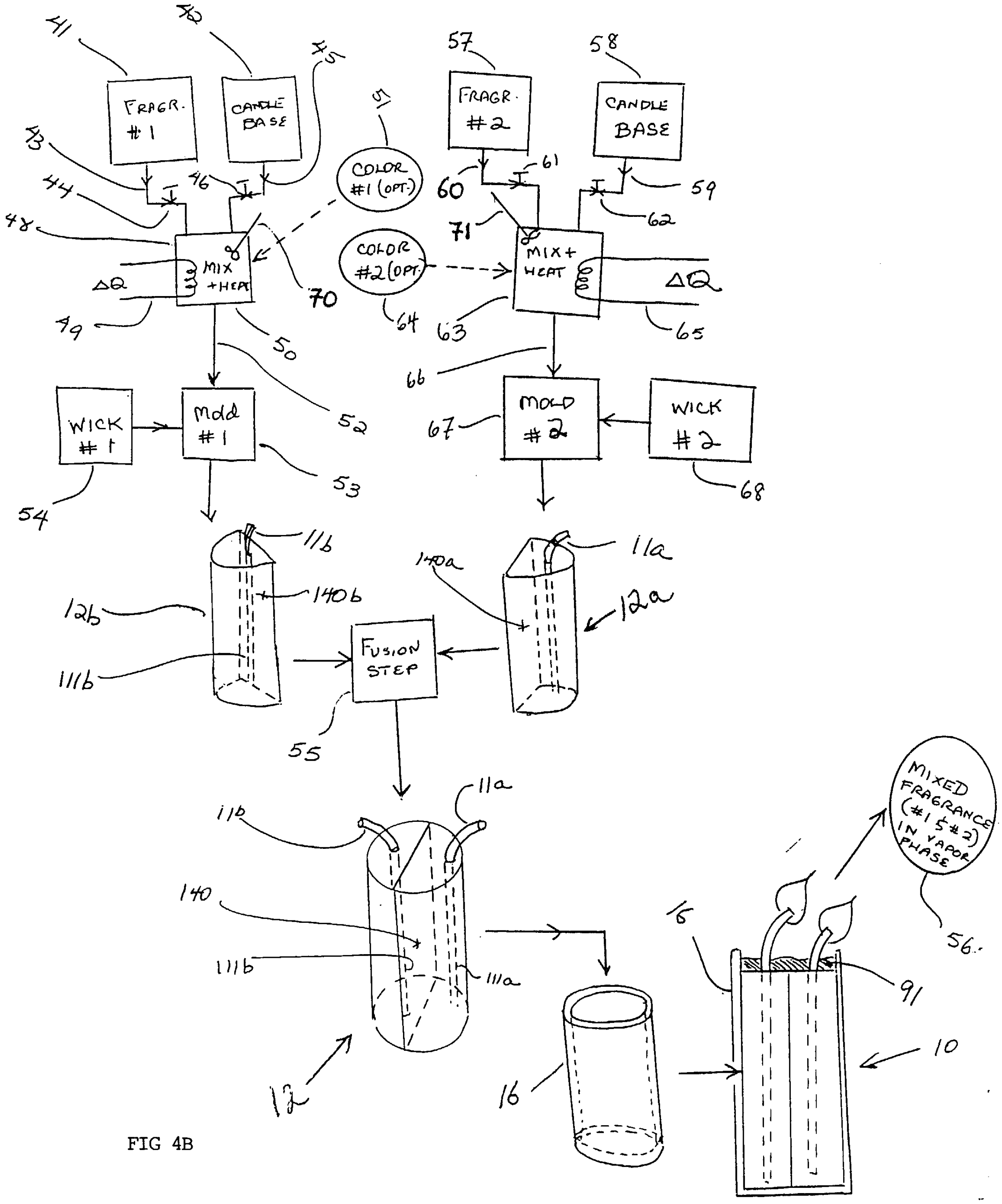
FRAGR. #1
CANDLE BASE
COLOR #1 (OPT.)
FRAGR. #2
CANDLE BASE
MIX +HEAT
COLOR #2 (OPT.)
MIX + HEAT
ΔQ
ΔQ
WICK #1
Mold #1
MOLD #2
WICK #2
FUSION STEP
MIXED FRAGRANCE (#1 & #2) IN VAPOR PHASE
41
42
45
51
57
58
43
46
44
48
70
60
61
59
62
71
64
63
65
49
50
66
52
67
54
53
68
11b
140b
12b
111b
11a
140a
12a
55
11a
11b
140
111b
111a
12
16
16
91
10
56

FIG 4B

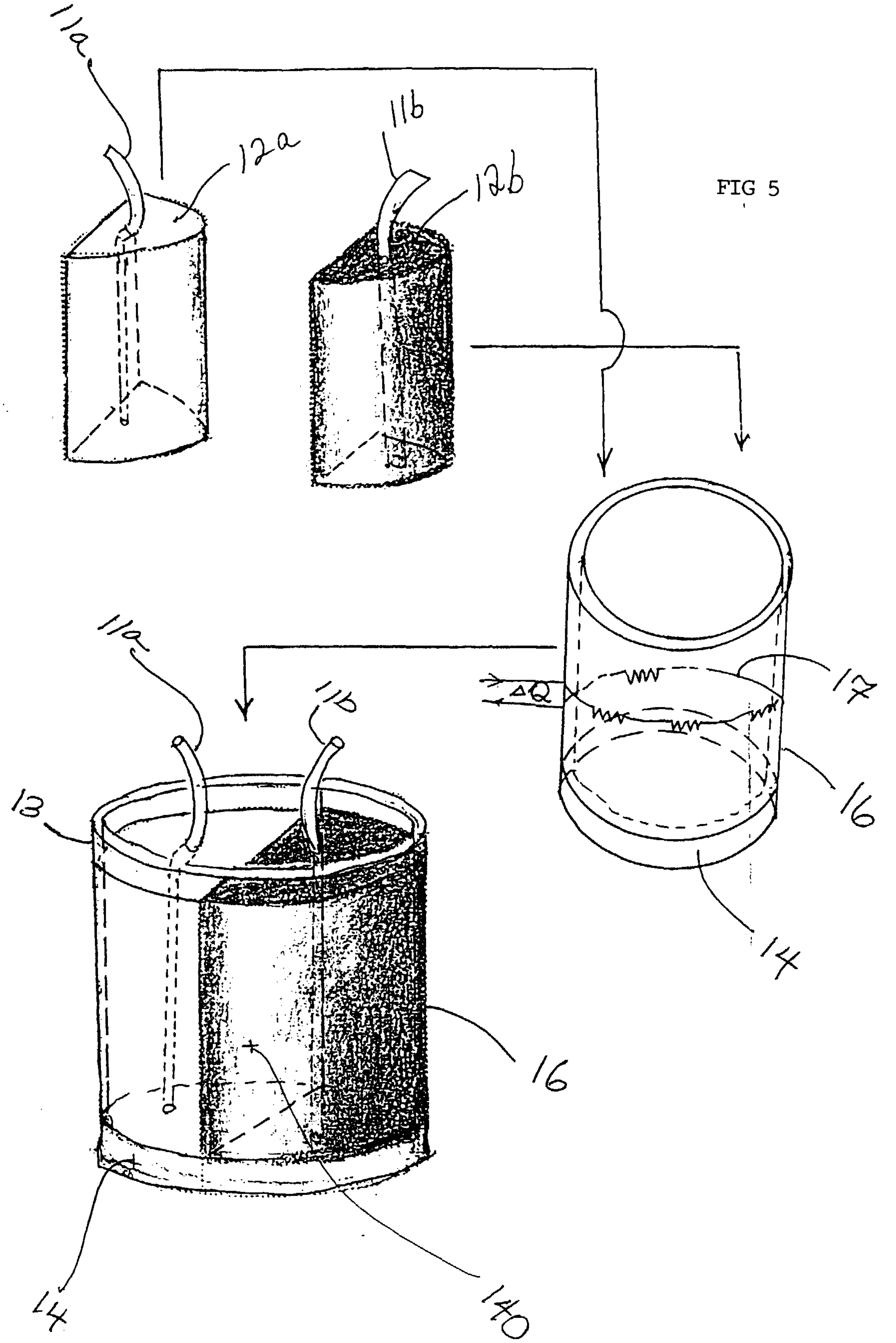


FIG 5

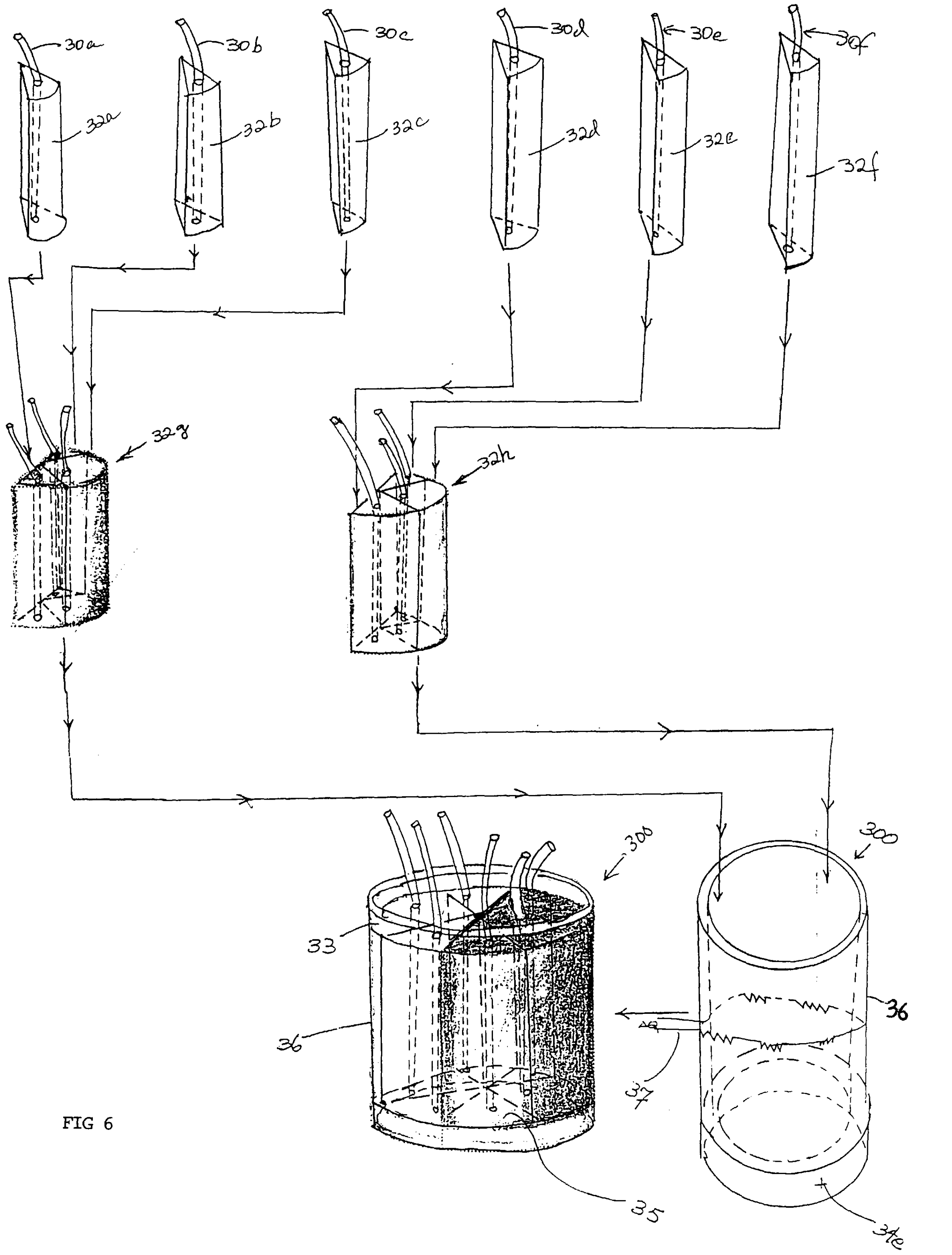
30a
30b
30c
30d
30e
30f
32a
32b
32c
32d
32e
32f
32g
32h
300
300
33
36
36
37
35

FIG 6

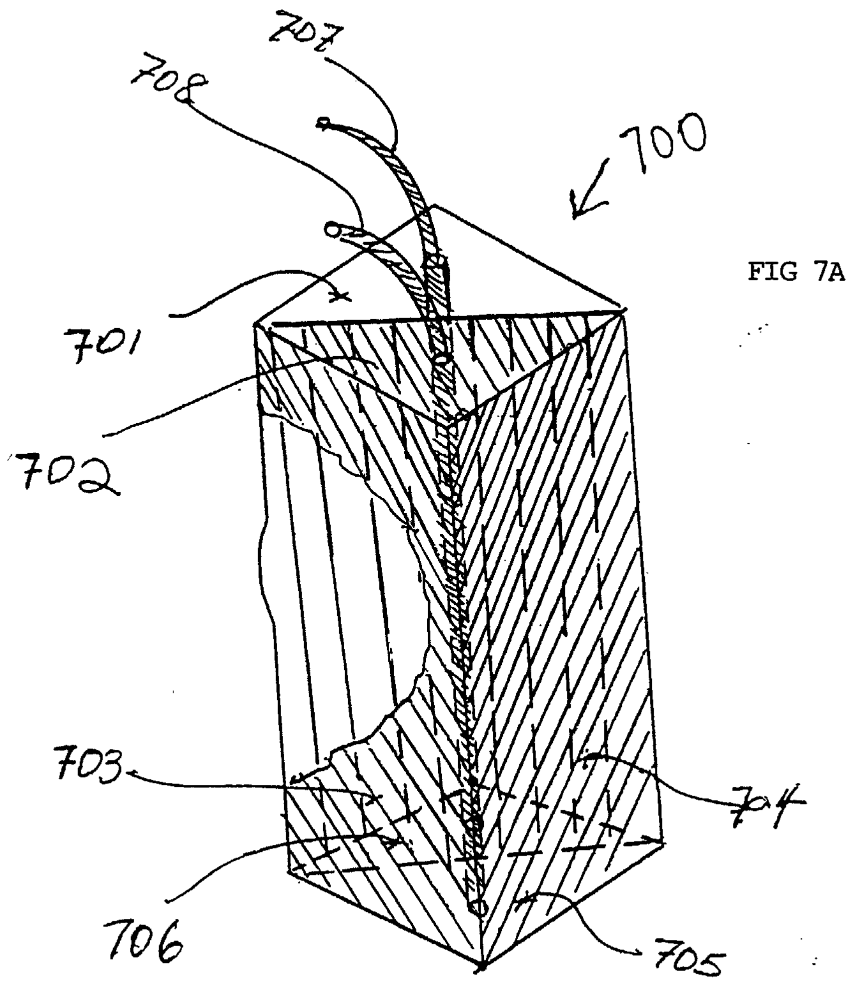


FIG 7A

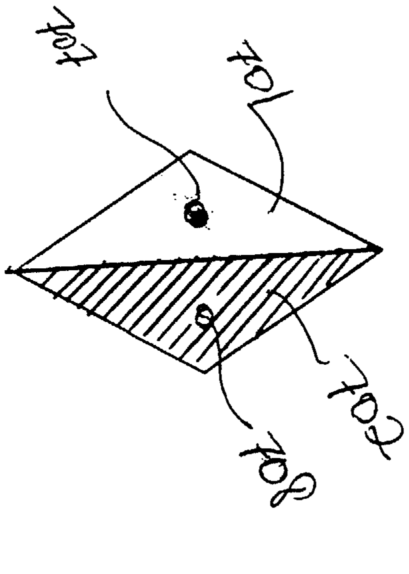


FIG 7B

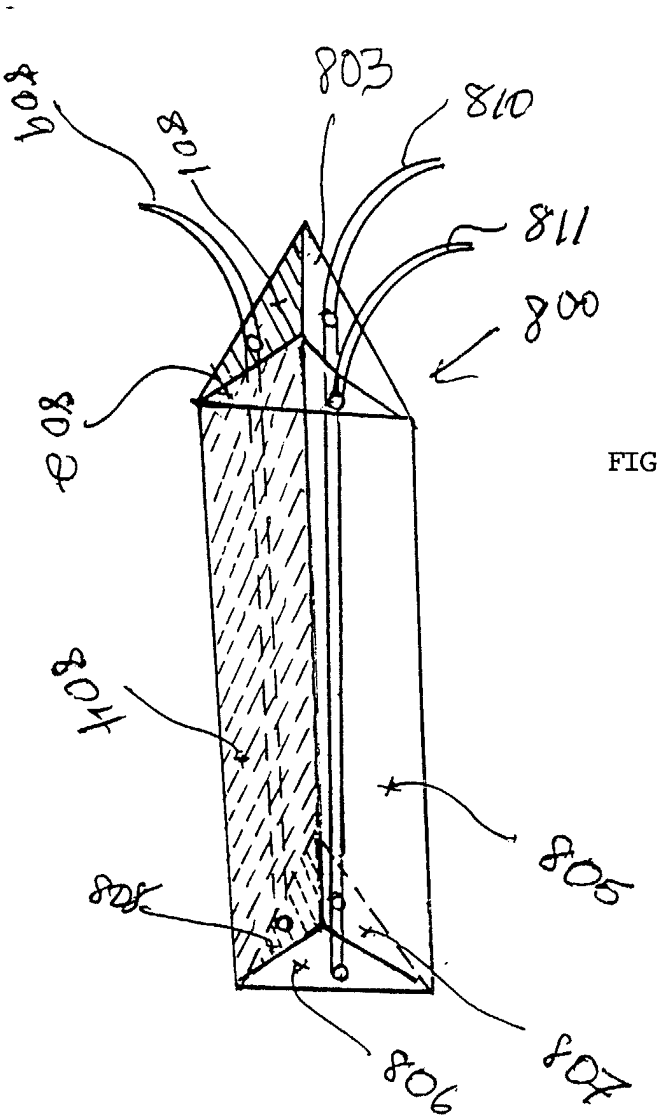
809
801
803
810
811
800
808
804
805
806
807

FIG 8A

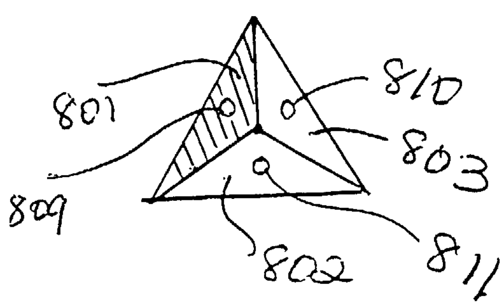
801
810
803
809
802
811

FIG 8B

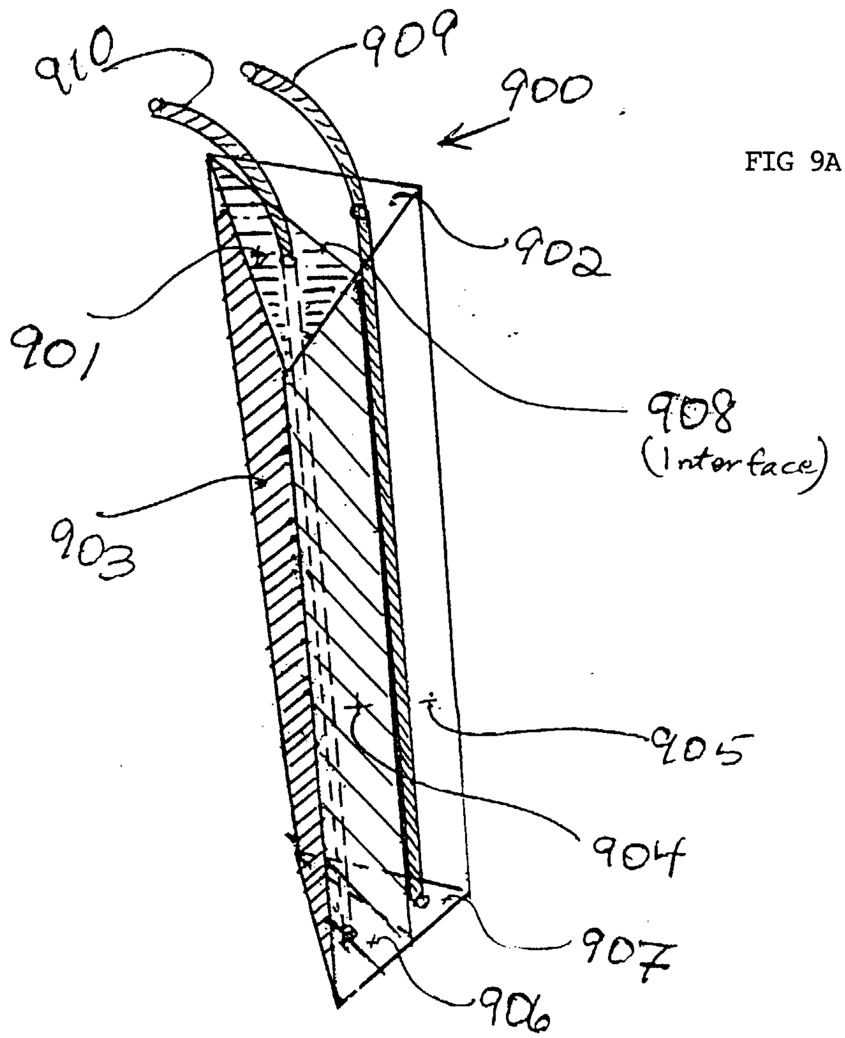
910
909
900
902
901
908
(Interface)
903
905
904
907
906

FIG 9A

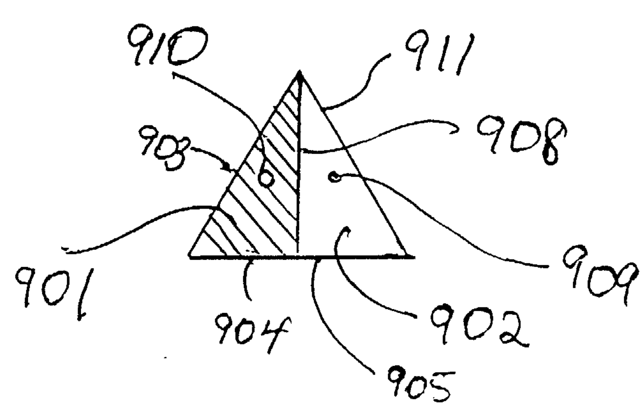
910
911
903
908
901
909
904
902
905

FIG 9B

FIG 10

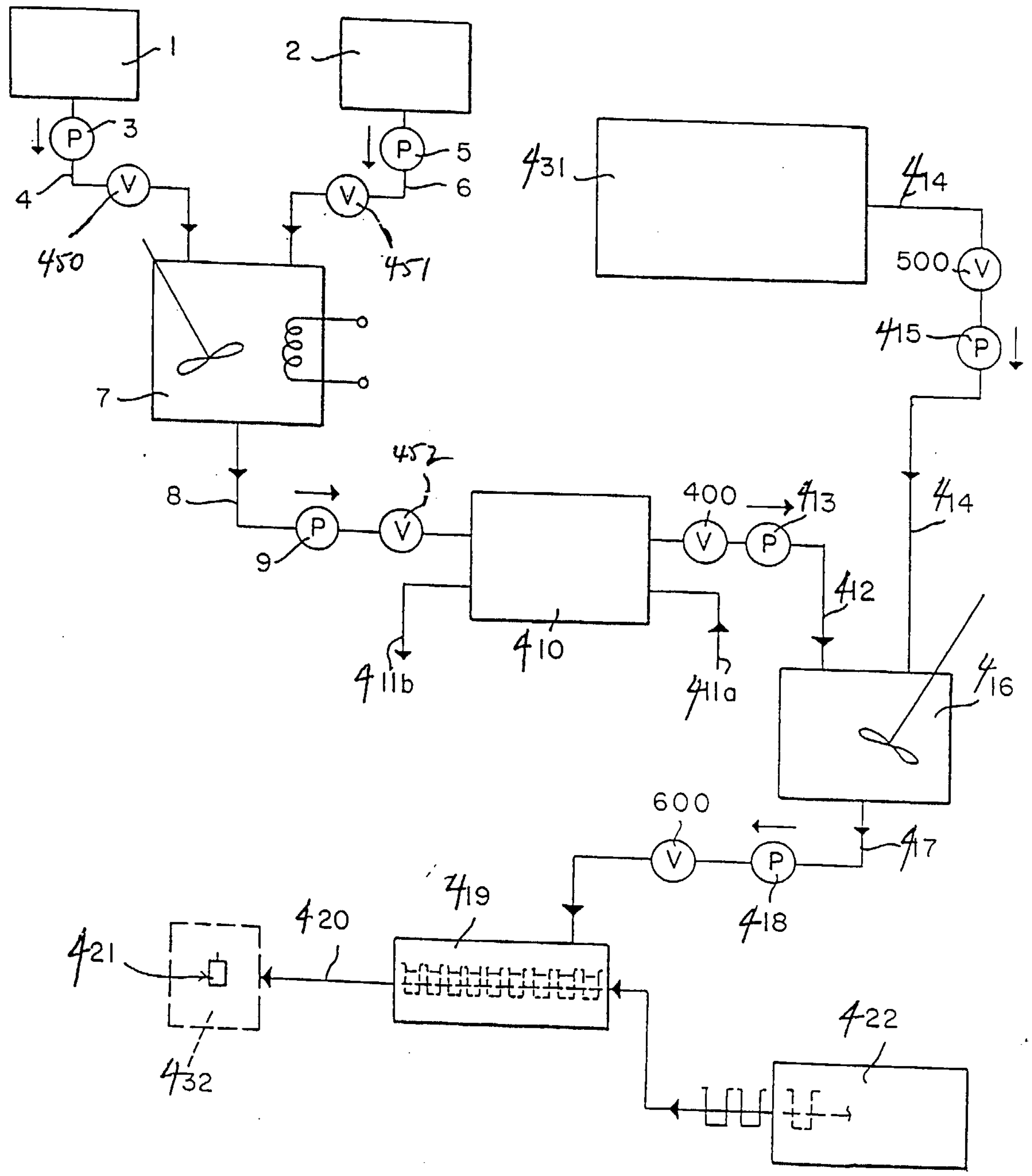


FIG 10A

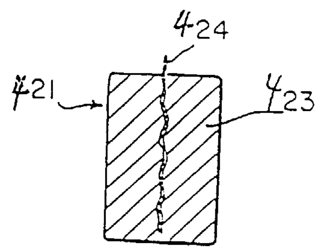

FIG 11

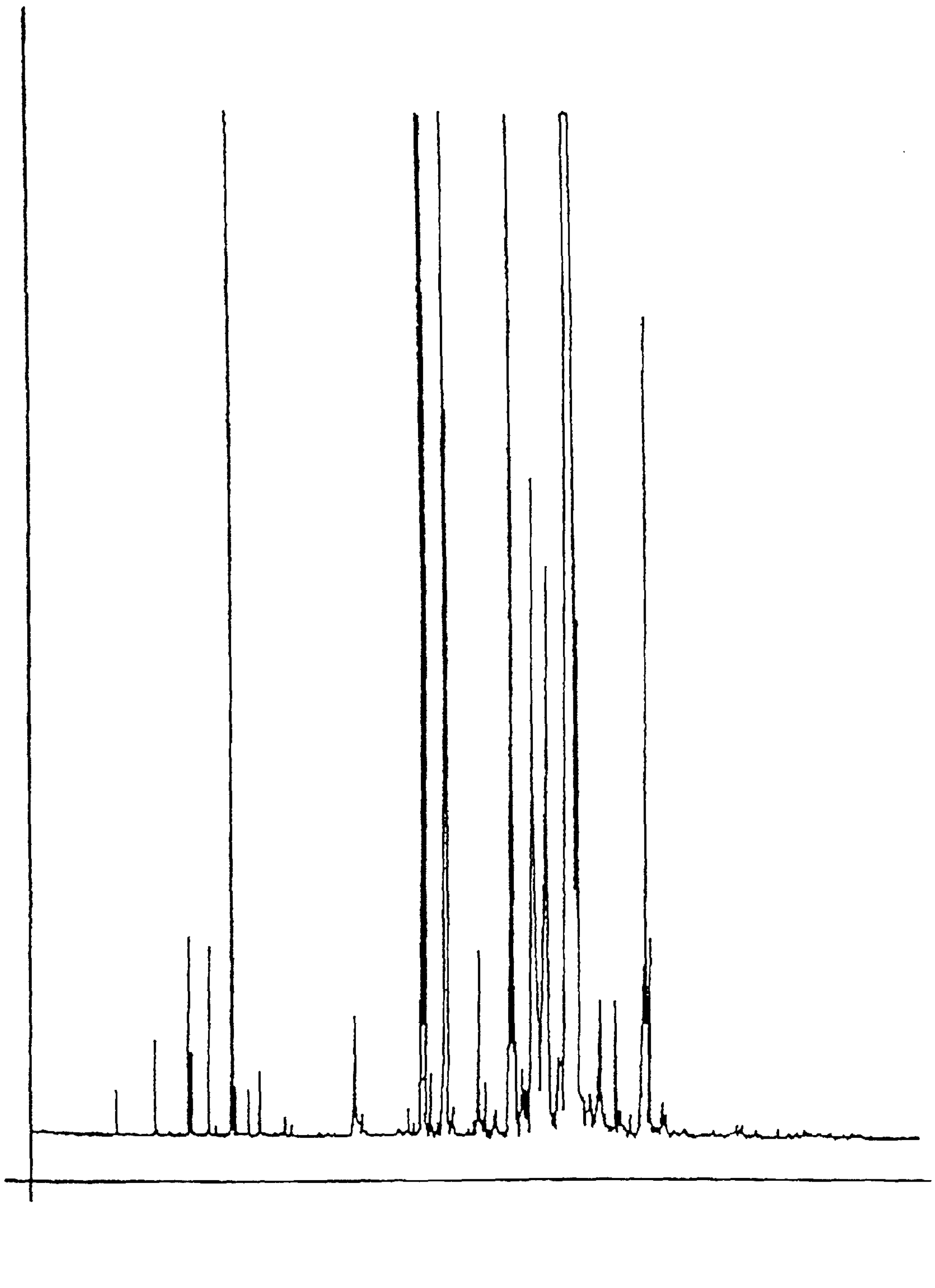

FIG 12

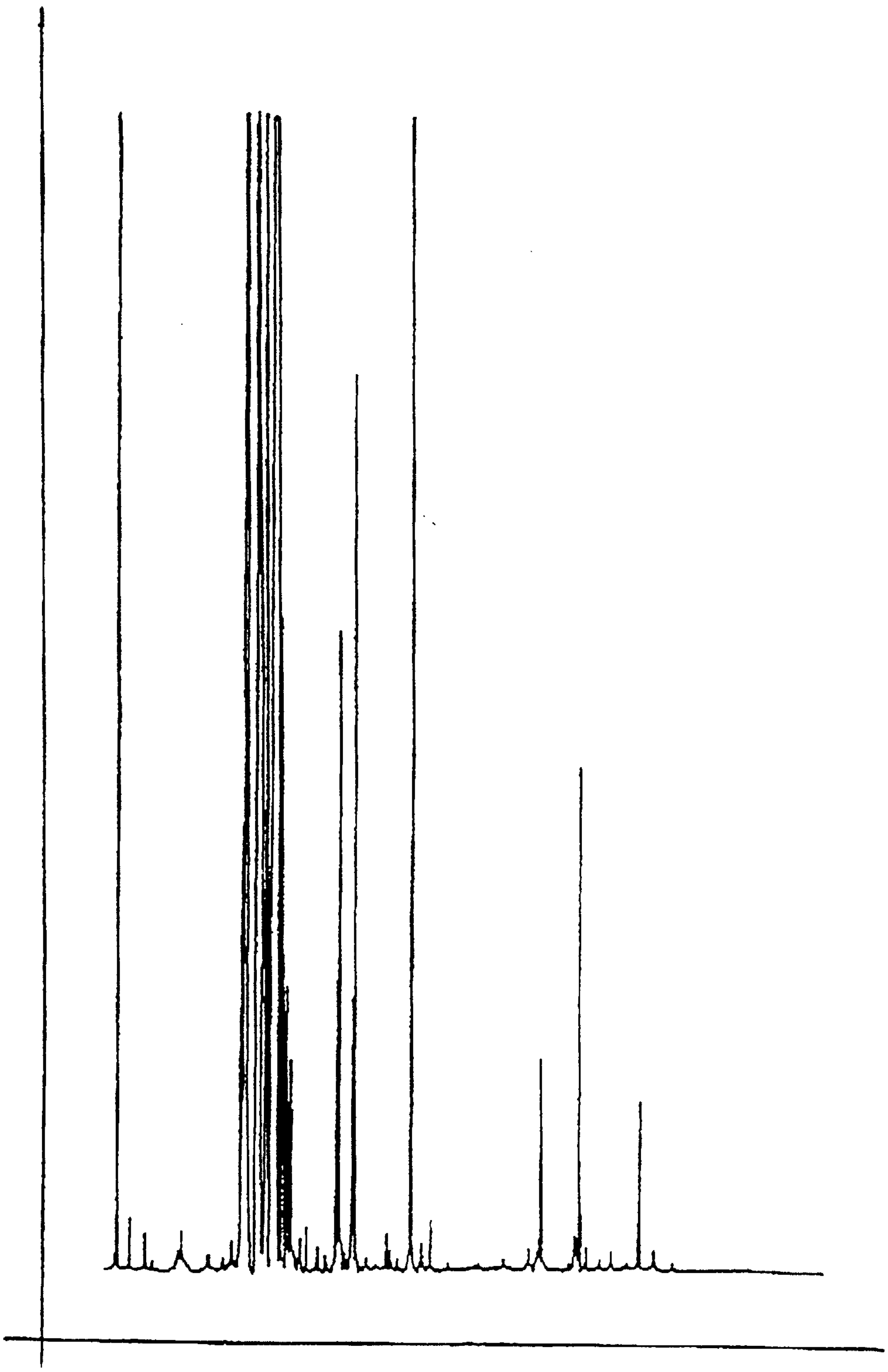

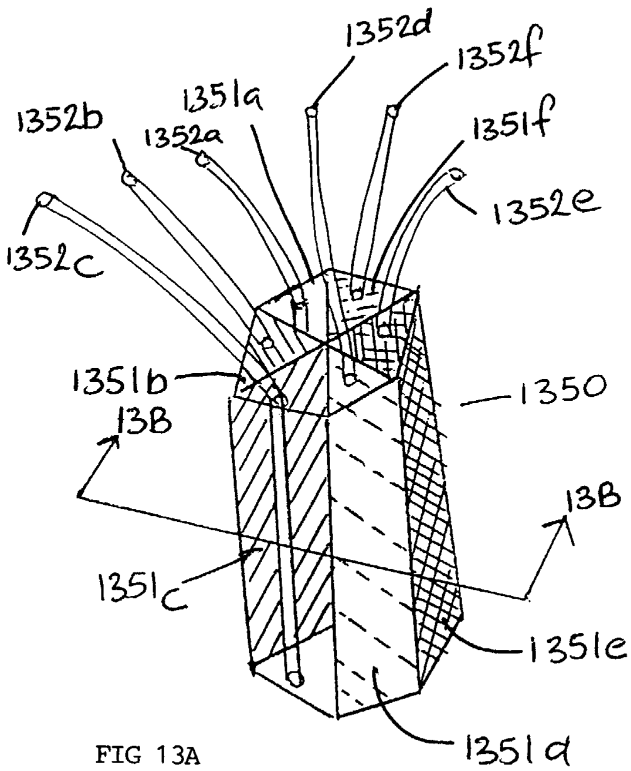


FIG 13A

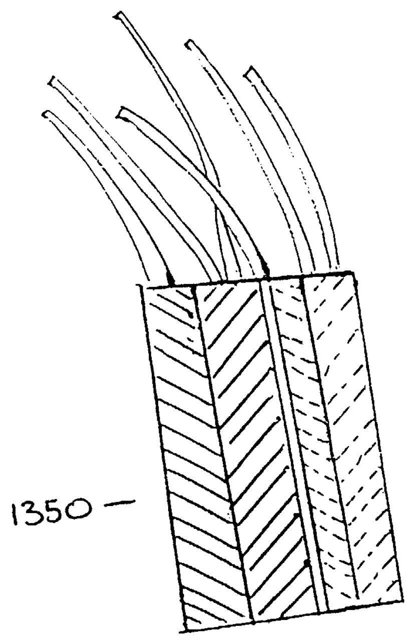


FIG 13C

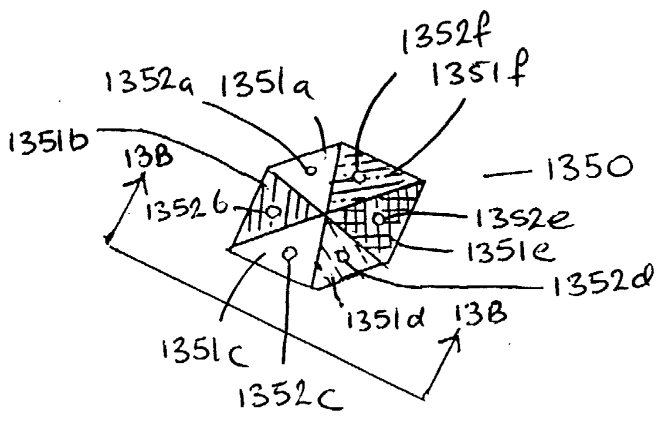


FIG 13B